# EUROPEAN PATENT SPECIFICATION

(11) **EP 2 012 794 B1**
(45) Date of publication and mention of the grant of the patent: **17.09.2014**
(21) Application number: 07755523.3
(22) Date of filing: 13.04.2007
(51) Int. Cl.: A61P 9/00, A61K 31/56, A61K 31/44

(54) **COMPOSITIONS AND INTRALUMINAL DEVICES FOR INHIBITING VASCULAR STENOSIS**
ZUSAMMENSETZUNGEN UND INTRALUMINALE VORRICHTUNG ZUR VERHINDERUNG VON GEFÄSSVERENGUNG
COMPOSITIONS ET DISPOSITIFS INTRALUMINAUX PERMETTANT D'INHIBER LA STENOSE VASCULAIRE

(30) Priority: 13.04.2006 US 792156 P
(43) Date of publication of application: 14.01.2009
(73) Proprietor: The Trustees of Columbia University in the City of New York, New York, New York 10027 (US)
(72) Inventor: MARKS, Andrew, Robert, Larchmont, NY 10538 (US)
(74) Representative: Lawrence, John
(86) International application number: PCT/US2007/009289
(87) International publication number: WO 2007/120897

(56) References cited:
- WO-A-03/009779
- WO-A-03/024183
- WO-A-2005/027973
- WO-A2-02/056790
- US-A1- 2003 022 911
- US-A1- 2003 033 007
- US-A1- 2004 213 757
- US-A1- 2006 240 014
- GIORDANO ET AL.: 'Rapamycin antagonizes NF-kappaB nuclear translocation activated by TNF-alpha in primary vascular smooth muscle cells and enhances apoptosis' 20 January 2006,
- ZAHRADKA ET AL.: 'Activation of MMP-2 in response to vascular injury is mediated by phosphatidylinositol 3-kinase-dependent expression of MT1-MMP' AM. J. PHYSIOL. HEART CIRC. PHYSIOL. vol. 287, 2004, pages H2861 - H2870
- SHAH ET AL: PNAS, vol. 105, no. 48, 2 December 2008 (2008-12-02), pages 19006-19011,

## Description

This application claims the benefit of U.S. Provisional Application No. 60/792,156, filed on April 13, 2006.

This patent disclosure contains material that is subject to copyright protection. The copyright owner has no objection to the facsimile reproduction by anyone of the patent document or the patent disclosure as it appears in the files of the U.S. Patent and Trademark Office or any other Patent Office, but otherwise reserves any and all copyright rights.

### BACKGROUND

Obese patients often have leptin resistance and elevated blood leptin levels or "hyperleptinemia". In addition, many obese patients suffer from type III or "insulin resistance" diabetes, and conversely, many patients with type II diabetes are obese. Obese and diabetic patients are particularly susceptible to vascular stenosis. As such, a high proportion of patients undergoing percutaneous transluminal coronary angioplasty ("PTCA") and cardiovascular stenting procedures to treat or ameliorate vascular stenosis, are obese and/or diabetic. Restenosis after PTCA or implantation of "bare" stents occurs in around 30% of patients. Recently, the use of drug-coated stents, such as rapamycin-coated stents, has decreased the rate of restenosis to around 10%. However, obese and diabetic patients are unusually resistant to the anti-restenotic activity of rapamycin. Accordingly, there is a need in the art to develop improved methods for treating and preventing vascular stenosis and restenosis in diabetic and obese patients.

### SUMMARY OF THE INVENTION

The present invention is based, in part, on the discovery that (a) high levels of leptin (or leptin plus insulin) may lead to rapamycin-resistance, (b) that the phosphatidylinositol 3-kinase ("PI3-kinase")/Akt signaling pathway is involved in this rapamycin-resistance, and (c) that a combination of a PI3-kinase inhibitor and rapamycin is more effective at inhibiting vascular smooth muscle cell proliferation and neointimal hyperplasia *in vivo*, than rapamycin alone.

Accordingly, the present invention provides compositions, and medical devices useful for the treatment and prevention of vascular stenosis and restenosis. The compositions, methods, and medical devices of the invention are particularly useful in diabetic and obese patients, and patients with metabolic syndrome, but may also be useful for any patient afflicted with, or at risk of developing, intimal hyperplasia, vascular stenosis and/or restenosis. The compositions of the invention comprise rapamycin, or a derivative thereof, in combination with (a) a PI3 kinase inhibitor, (b) a leptin inhibitor, or (c) both a PI3 kinase inhibitor and a leptin inhibitor. Such compositions may be used to treat or prevent vascular stenosis and restenosis. For example, such compositions may be used to coat an intraluminal device used to treat or prevent vascular stenosis or restenosis, such as a vascular stent.

In one embodiment, the present invention provides a composition for use in the treatment or prevention of vascular stenosis or restenosis comprising a therapeutically effective amount of (i) rapamycin or a rapamycin derivative, and (ii) a PI3-kinase inhibitor, in specified amounts

According to the above embodiments, the compositions of the invention may comprise a rapamycin derivative. In one embodiment, the rapamycin derivative is selected from the group consisting of: everolimus (RAD-001), temsirolimus (CCI-779), AP23573, ABT-578, 42-epi-(tetrazoylyl) rapamycin, 30-demethoxy rapamycin, a rapamycin 29-enol, a tetrazole-containing rapamycin derivative, a mono-ester derivative of rapamycin, a di-ester derivative of rapamycin, a 27-oxime derivative of rapamycin, a 42-oxo derivative of rapamycin, a bicyclic derivative of rapamycin, a rapamycin dimer, a silyl ether derivative of rapamycin, an arylsulfonate derivative of rapamycin, and a sulfamate derivative of rapamycin, or any other rapamycin derivative described in the Detailed Description.

Also according to the above embodiments, the compositions of the invention may comprise a PI3-kinase inhibitor. In one embodiment, the PI3-kinase inhibitor is selected from the group consisting of: wortmannin, wortmannin analogue 2, wortmannin analogue 3, wortmannin analogue 4, wortmannin analogue 5, wortmannin analogue 6, wortmannin analogue 7, wortmannin analogue 8, (+)-halenaquinol, (+)-halenaquinone, (+)-xestoquinone, viridian, viridol, demethpxyviridin, demethoxyviridol, wortmannolone, noelaquinone, 2-(4-morpholinyl)-8-phenyl-4H-1-benzopyran-4-one, LY294002", quercitin, myricetin, and staurosporine, and derivatives thereof, or is another PI3-kinase inhibitor as described in the Detailed Description. In one preferred embodiment, the PI3-kinase inhibitor is wortmannin, or a wortmannin derivative. In another preferred embodiment, the PI3-kinase inhibitor is LY294002, or a derivative thereof.

In one embodiment, the present invention provides an intraluminal device for use in the treatment or prevention of vascular stenosis or restenosis, wherein the intraluminal device is impregnated with, and configured to release, a therapeutically effective amount of a composition comprising (i) rapamycin or a derivative thereof, and (ii) a PI3-kinase inhibitor. In another embodiment, the present invention provides an intraluminal device for use in the treatment or prevention of vascular stenosis or restenosis, wherein the intraluminal device impregnated with, and configured to release, a therapeutically effective amount of a composition comprising (i) rapamycin or a derivative thereof, and (ii) a leptin inhibitor. In yet another embodiment, the present invention provides an intraluminal device for use in the treatment or prevention of vascular stenosis or restenosis, wherein the intraluminal device is impregnated with, and configured to release, a therapeutically effective amount of a composition comprising (i) rapamycin or a derivative thereof, (ii) a PI3-kinase inhibitor, and (iii) a leptin inhibitor. In each of these embodiments, it is preferred that the rapamycin derivatives, PI3-kinase inhibitors, and leptin inhibitors, are selected from those described above or described in the Detailed Description.

In certain embodiments, the intraluminal devices of the invention may be stents, shunts, catheters, arterio-venous grafts, by-pass grafts, vascular grafts, or balloons for use in balloon angioplasty. In preferred embodiments, the intraluminal devices of the invention are stents. Thus, the invention provides drug-eluting stents that contain, are coated with, or otherwise are configured to release the rapamycin-containing combination compositions of the invention. For example, in one preferred embodiment, the invention provides a stent having a composition of the invention coated onto its surface. In another preferred embodiment, the invention provides a stent having a composition of the invention within micropores on its surface. In various embodiments, compositions of the invention are incorporated into a polymer which is used to coat a stent, or which is added to micrpores in a stent, or a polymer that is used to form the stent body.

In one embodiment, the present invention provides a method for treating or preventing vascular stenosis or restenosis in a subject in need thereof comprising administering to the subject a composition of the invention as described above, or an intraluminal device as described above. In preferred embodiments, these methods of treatment (or prevention) are performed concurrently with the subject undergoing a percutaneous transluminal coronary angioplasty ("PTCA") procedure, i.e. a balloon angioplasty procedure. In other preferred embodiments, these methods of treatment (or prevention) are performed subsequent to the subject undergoing a PTCA procedure. The methods of the invention may be used to treat or prevent vascular stenosis or restenosis in any subject, however, in preferred embodiments the subjects are mammals, and in more preferred embodiments, the subjects are humans. In even more preferred embodiments, the subjects are humans who are overweight, obese, diabetic, or have metabolic syndrome, or are at risk of developing one of these conditions.

### BRIEF DESCRIPTION OF THE FIGURES

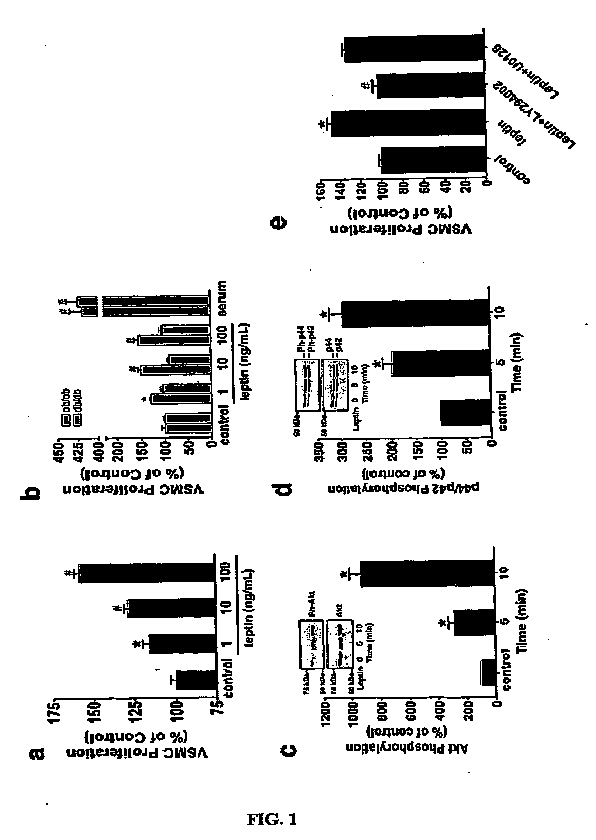

Figure 1 provides data showing that leptin activates mTOR signaling in cultured murine vascular smooth muscle cells (VSMCs) and stimulates VSMC proliferation in a leptin receptor- and PI3K-dependent fashion. Panels (a) (b) and (e) show effects of the indictaed agents on VSMC proliferation. In panels (a) and (b) serum starved primary aortic VSMCs from wild type ("WT"), ob/ob, or db/db mice were stimulated with the indicated concentrations of leptin or serum. In panel (b) the left-hand bar in each pair of bars is data from ob/ob mice, and the right-hand bar in each pair of bars is data from db/db mice. The * symbol indicates a P value of <0.05, the # symbol indicates a P value of <0.001, compared to control. Panels (c) and (d) show Akt and MAPK phosphorylation after 0, 5, and 10 minutes of leptin treatment The * symbol indicates a P value <0.05 compared with control. Panel (e) shows the effects of LY294002 and U0126 in a VSMC proliferation assay. The * symbol indicates a P value of <0.001 compared to the control. The # symbol indicates a P value of <0.001 compared to leptin alone.

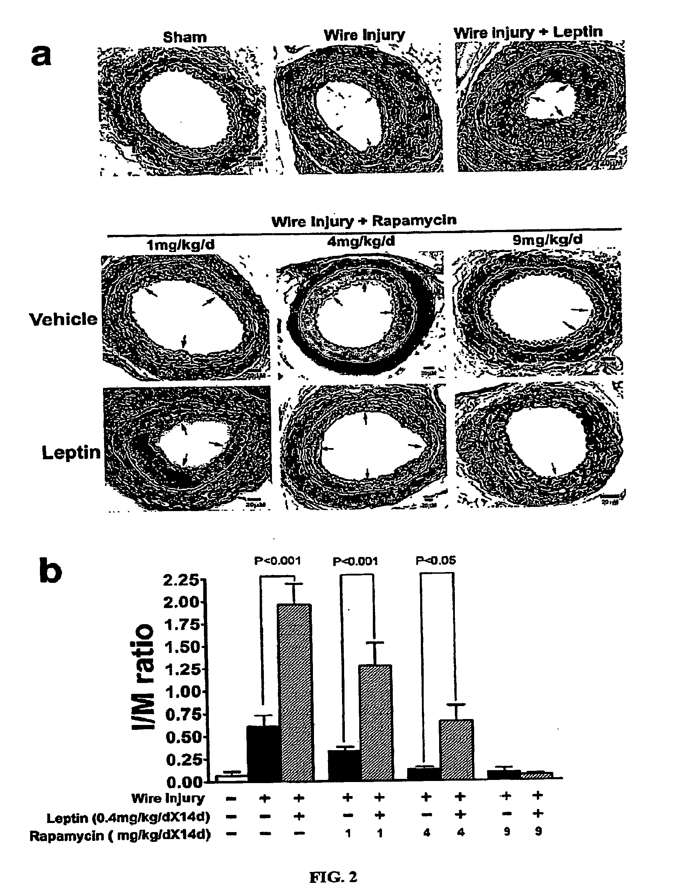

Figure 2 provides data showing that leptin-induced neointimal hyperplasia after arterial injury in mice is resistant to sirolimus inhibition. Panel (a) data from a femoral artery injury model. Micrographs of neointimal formation (indictaed by the arrows) in representative injured femoral arteries in the presence or absence of leptin and rapamycin are indicated. Cross-sections were stained with elastic-Van Gieson to highlight the elastic laminae in the tunica media (black rings) and collagen in the tunica adventitia. Panel (b) shows intima:media (I/M) ratios measured from femoral arteries. The white bars show sham injury. The solid black bars show injury without leptin. The striped bars show injury with leptin. The P values after Tukey's test are indicated above the brackets.

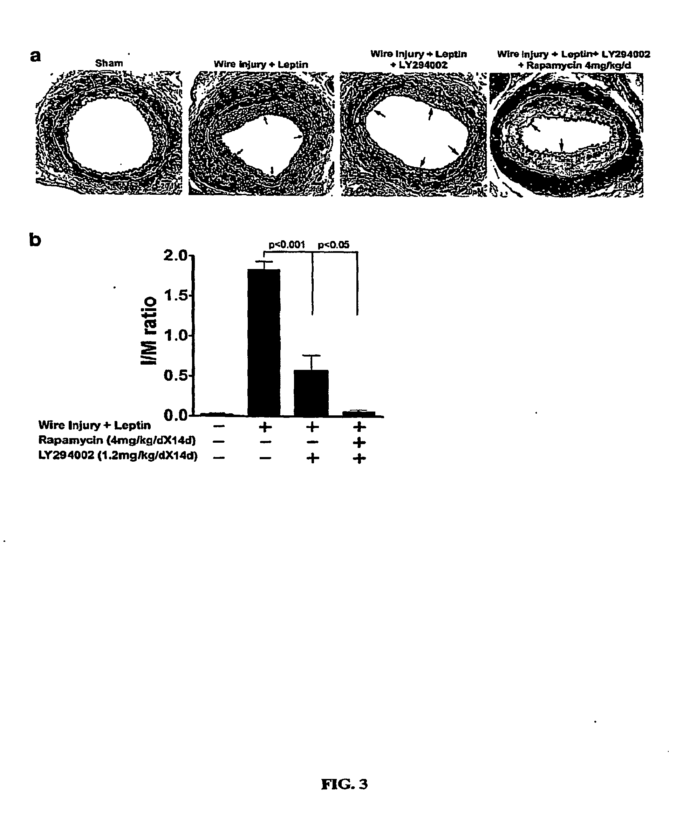

Figure 3 provides data showing that a combination of a PI3K inhibitor (LY294002) and an mTOR inhibitor (sirolimus) inhibits leptin-induced neointimal hyperplasia in mice. Panel (a) shows data from femoral artery injury studies. Micrographs of leptin-induced neointimal formation (arrows) in injured femoral arteries after treatment with LY294002 and/or rapamycin are illustrated. Elastic-van Gieson staining was performed to highlight elastic laminae (black rings) and collagen. Panel (b) shows I/M ratios. Tukey's P values are provided over the brackets.

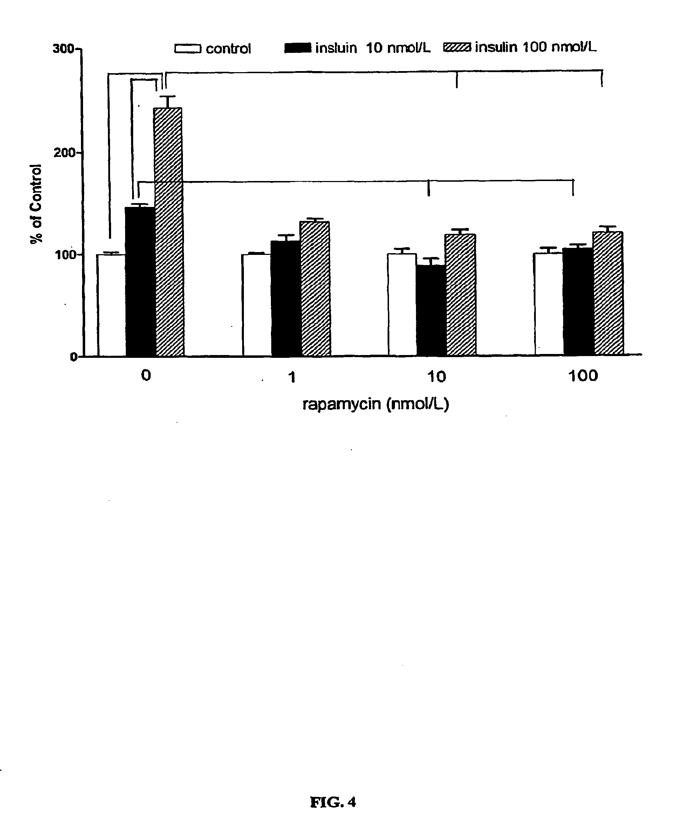

Figure 4 is a bar graph showing rapamycin inhibition of insulin-stimulated VSMC proliferation n the presence or absence of insulin (10 or 100 nmol/L) and/or rapamycin (0, 1, 10, or 100 nmol/L). Brackets indicate significance differences (P < 0.01).

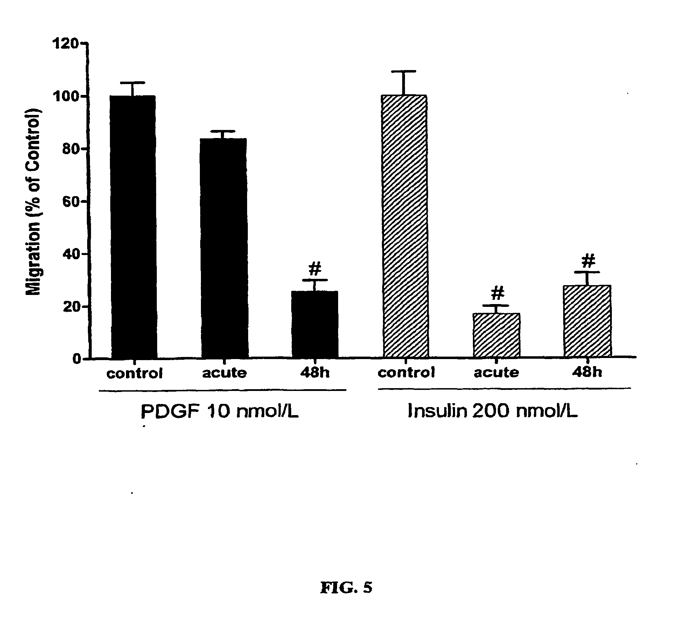

Figure 5 provides a bar chart that depicts migration of VSMCs toward insulin in a modified Boyden chamber, and shows inhibition of VSMC migration by rapamycin on. Effects of acute and 48h exposure of VSMCs to rapamycin (RPM, 100 nmol/L) toward PDGF (10 nmol/mL) and insulin (200 nmol/L) are shown. The # symbol indicates value is significant compared to control group, P<0.01.

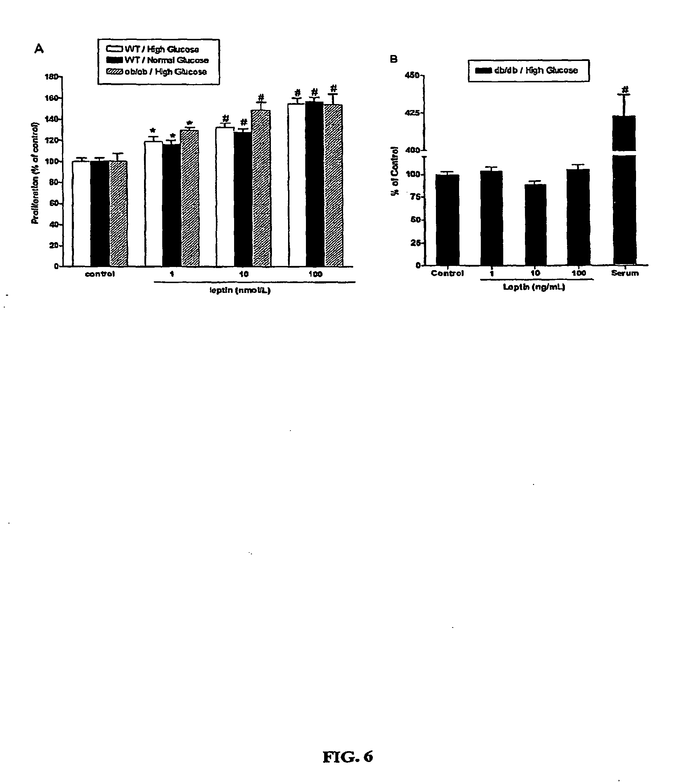

Figure 6 provides bar charts illustrating that leptin stimulates VSMC proliferation in a dose-dependent manner in the presence of both high and low glucose concentrations. Panel A provides data from wild type mice with high glucose levels (450 mg/dL) or normal glucose levels (125 mg/dL) and from leptin deficient *ob*/*ob* mice. Panel B provides data from leptin receptor defective db/db mice, and shows that leptin has no effect on VSMC proliferation in cells in *dbldb* mice, compared with 10% of foetal bovine serum (serum). The * symbol indicates a significance value of P<0.05 compared to control samples. The # symbol indicates a significance value of P<0.01 compared to control samples.

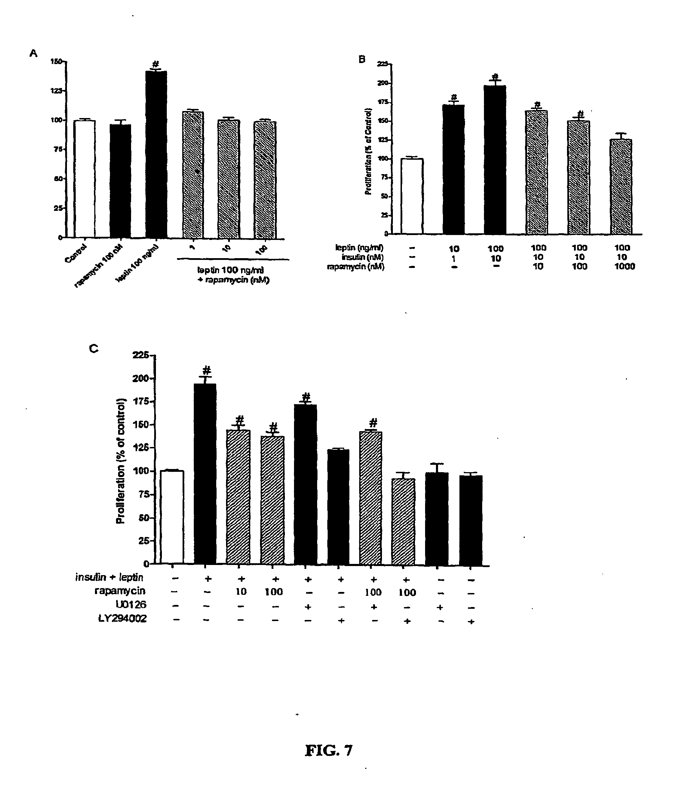

Figure 7 provides bar graphs showing that rapamycin inhibits leptin-stimulated VSMC proliferation but not VSMC proliferation stimulated by a combination of insulin and leptin. In panel C, VSMCs were also treated with U0126 (a specific MEK1/2 inhibitor), or LY294002, (a specific PI3K inhibitor). The # symbol indicates that the value was statistically significant compared with the control group, P<0.01.

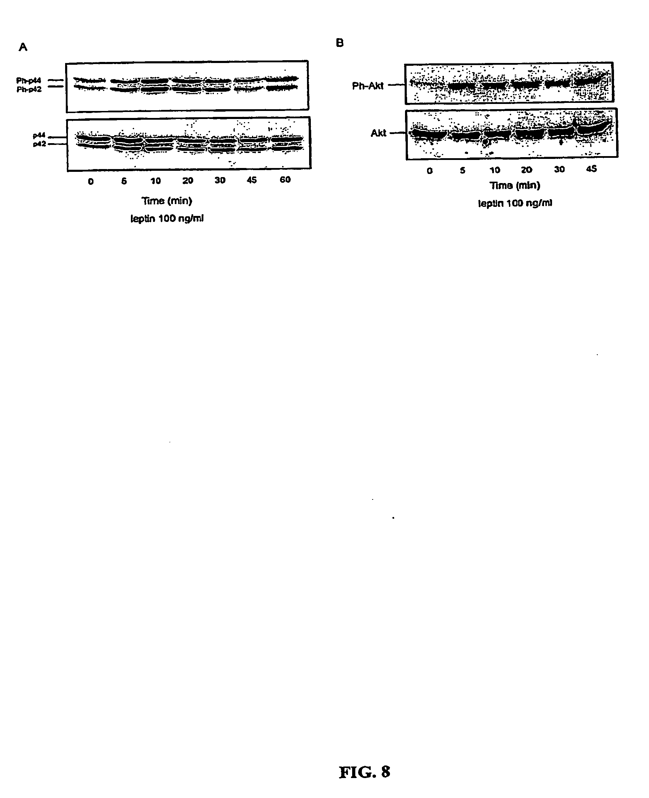

Figure 8 provides Western blotting data showing that leptin stimulates phosphorylation of ERK1/2 and Akt. In panel A, whole cell extracts were analyzed by immunoblotting using an anti-phosphorylated-ERK1/2 antibody (upper panel) or anti-ERK1/2 (lower panel). In panel B, the whole cell extracts were analyzed by immnoblotting using an anti-phosphorylated-Akt (Ser437) antibody (upper panel) or anti-Akt antibody (lower panel).

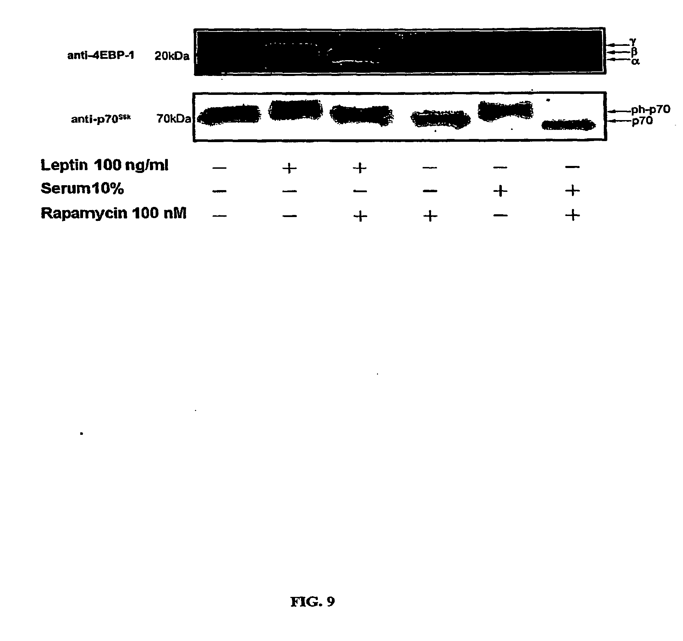

Figure 9 provides Western blotting data showing that rapamycin completely dephosphorylates 4E-BP1 and p70^{S6K}, proteins that are phosphorylated by leptin and serum in the absence of rapamycin. Whole cell extracts were analyzed by western blot with anti-4E-BP1 antibody (upper panel) and anti-P70^{S6K} (lower panel) antibodies. The mobility of phosphorylated 4E-BP1 and P70^{S6K} proteins is reduced on the SDS-gel.

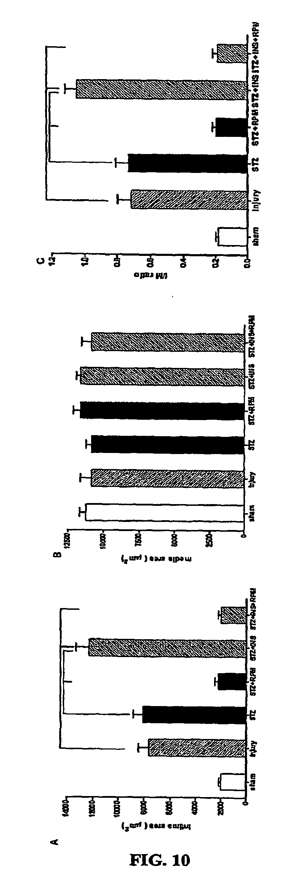

Figure 10 provides bar graphs illustrating that rapamycin inhibits neointimal formation in a femoral artery model following wire injury. Wire injury lesions in Streptozotocin-induced diabetic mice were divided into five groups, sham operation group (sham), hyperglycemic without other treatment ("STZ" - indicated treatment with streptozotocin to induce hyperglycemia), hyperglycemic with insulin treatment (STZ + INS), hyperglycemic with rapamycin treatment (STZ + RPM), and hyperglycemic with insulin and rapamycin treatment (STZ + INS + RPM). The intimal area (panel A), media area (panel B), and intima:media ratio (panel C) were measured. The # symbol indicates that values were significant to P<0.01 when compared with the sham group.

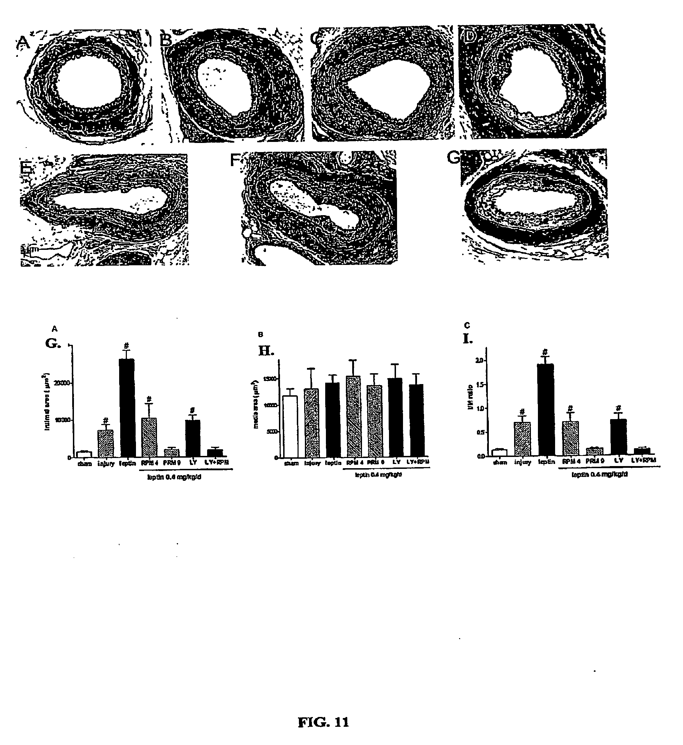

Figure 11 provides bar graphs showing that leptin enhances neointimal hyperplasia in a wire-injured femoral artery model, and that this effect is partially inhibited by rapamycin at 4 mg/kg/d. Histologic sections are shown in the top panels (A-G). Mice that underwent femoral artery surgery were divided into seven groups: (A) sham, (B) wire injury, (C) wire injury with leptin 0.4 mg/kg/d, (D) wire injury with leptin 0.4 mg/kg/d and rapamycin 4 mg/kg/d, (E) wire injury with leptin 0.4 mg/kg/d and rapamycin 9 mg/kg/d, (F) wire injury with leptin 0.4 mg/kg/d and LY294002 1.2 mg/kg/d, and (G) wire injury with leptin 0.4 mg/kg/d and rapamycin 4 mg/kg/d plus LY294002 1.2 mg/kg/d. Representative micrographs from the femoral arteries of groups A-G are shown in panels A-G. Bars represent 10 µm. The lower panels show the intimal area (panel H), media area (paneal I), and intima:media ratio (panel J). The # symbol indicates statistical significance compared with the sham group at the P<0.01 level.

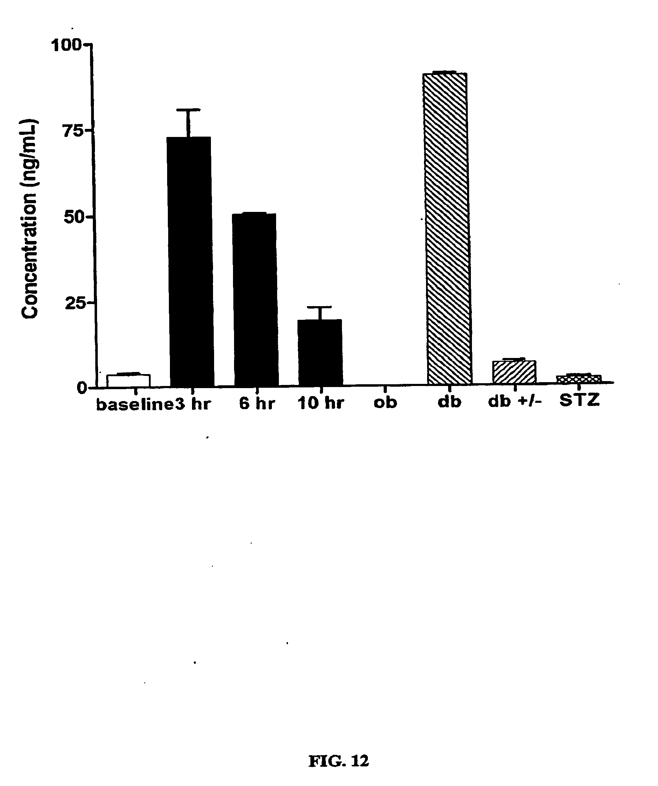

Figure 12 provides a bar graph of showing serum leptin concentrations (ng/mL) in untreated normal mice (clear bar) mice that had been treated by intraperitoneal injection of leptin at 0.4 mg/kg (black bars), and ob/ob, db/db, db+/- and STZ-treated mice (cross-hatched bars). In the leptin-treated group, blood samples were collected 3, 6 and 10 hours after injection.

### DETAILED DESCRIPTION OF THE INVENTION

The present invention provides compositions, methods, and medical devices, useful for the treatment and prevention of vascular stenosis and restenosis in diabetic and obese subjects and subjects who have metabolic syndrome, and also in other subjects who are suffering from, or at risk of developing, hyperproliferative cardiovascular diseases.

As used herein the term "vascular stenosis" refers to narrowing of the lumen of a blood vessel or, conversely, thickening of the blood vessel wall. Vascular stenosis is generally caused by over-proliferation of cells of the blood vessel wall and/or inflammatory processes in the blood vessel wall. For example, "intimal hyperplasia" is thickening of the Tunica intima caused by overproliferation of the intimal cells, and is a major contributor to the process of vascular stenosis. Examples of conditions that cause, or are associated with vascular stenosis include, but are not limited to, atherosclerosis, coronary artery disease, obesity, metabolic sybndrome, peripheral artery occlusive disease, peripheral vascular disease, peripheral artery disease, carotid artery stenosis, and hyperproliferative vascular disease.

The term "restenosis" refers to a recurrence of vascular stenosis that occurs after treatment of a prior stenosis. As such, the term "restenosis" is encompassed by the term "stenosis", and unless stated otherwise, the term "stenosis" should be construed so as to encompass "restenosis. The term "neointimal hyperplasia" refers to "new" intimal hyperplasia, such as intimal hyperplasia occurring a new after treatment of a pre-existing intimal hyperplasia. As such, the term "neointimal hyperplasia" is encompassed by the term "intimal hyperplasia", and unless stated otherwise, the term "intimal hyperplasia" should be construed so as to encompass "neointimal hyperplasia". Importantly, while the compositions, methods, and medical devices of the invention are particularly well-suited to the treatment and/or prevention of restenosis an neointimal hyperplasia, they are also useful for the treatment and/or prevention of vascular stenosis and intimal hyperplasia more generally.

### Compositions of the Present Invention

The present invention provides compositions comprising rapamycin in combination with a PI3 kinase inhibitor. The present invention also provides compositions comprising derivatives of rapamycin, and the leptin inhibitors and PI3 kinase inhibitors described herein.

As used herein, the term "derivative" means a compound whose structure is the same as, or similar to, that of the named compound, but which has some chemical or physical modification, such as replacement of one or more individual atoms with a different atom or with a different functional group, addition of extra side groups, or removal of side groups. Also within the definition of "derivatives" as used herein, are polymerized forms of the named compound, copolymers of the named compound, enantiomers, diastereomers, tautomers, pharmaceutically acceptable salts, hydrates, solvates, and complexes of the named compound, and prodrugs of the named compound — i.e. versions of the drug that are converted to their active form within the body. In addition, in embodiments where the active agents of the invention are proteins or peptides (including antibodies), the term "derivative" includes homologues or fragments of those proteins or peptides, peptidomimetic agents, and humanized or fully human derivatives of those proteins or peptides. "Derivatives" of the invention are useful for inhibiting one or more of (a) VSMC proliferation, (b) intimal hyperplasia, (c) vascular inflammation, (d) vascular stenosis, and (e) vascular restenosis.

The compositions of the present invention may be delivered intraluminally to the vicinity of a stenotic lesion (including a restenotic lesion) in a subject in need thereof. Further description of how the agents may be delivered intraluminally using intraluminal devices is provided below.

In addition to the active agents listed above (namely rapamycin or rapamycin derivatives, PI3-kinase inhibitors and leptin inhibitors), the compositions of the invention may comprise one or more pharmaceutically acceptable solvents (such as aqueous or nonaqueous solvents), diluents, carriers, excipients, surfactants, adjuvants, preservatives, stabilizers, wetting agents, emulsifying agents, antibacterial agents, antifungal agents, sugars, salts, agents that promote sustained release of the active compounds, agents that facilitate or limit absorption, and the like.

One of skill in the art can readily select suitable agents for inclusion in the compositions of the invention, for example by consulting "Remington's Pharmaceutical Sciences", Gennaro, A.R., 18th Edition, Mack Publishing Co., Easton, PA.

The compositions of the invention may also include one or more therapeutically effective agents in addition to the rapamycin or rapamycin derivatives, PI3-kinase inhibitors and/or leptin inhibitors, such as other agents that are useful for inhibiting (a) VSMC proliferation, (b) intimal hyperplasia, (c) vascular inflammation, (d) vascular stenosis, or (e) vascular restenosis.

### Rapamycin and Derivatives Thereof

In certain aspects, the present invention provides compositions comprising rapamycin (also referred to as sirolimus and rapamune) and/or derivatives thereof, in combination with (a) a leptin inhibitor, (b) a PI3 kinase inhibitor, or (c) both a leptin inhibitor and a PI3 kinase inhibitor. In other aspects the present invention provides methods of treating or preventing vascular stenosis or vascular restenosis comprising administration of such compositions and intraluminal devices coated with such compositions.

Rapamycin is a macrolide antibiotic produced by the bacterium *Streptomyces hygroscopicus*. In addition to being an antibiotic, rapamycin also has antiproliferative, immunosuppressive, and anti-inflammatory properties. Rapamycin binds to its intracellular receptor, FKBP12, and inhibits the "mammalian Target of Rapamycin" (mTOR), which is a phosphatidylinositol-related kinase that regulates cell growth and proliferation in response to mitogens and nutrients through regulation of transcription, translation, and cell cycle progression For example, rapamycin inhibits proliferation of vascular smooth muscle cells, and inhibits intimal, hyperplasia. These properties make rapamycin useful for the treatment or restenosis. For example, the commercially available rapamycin-eluting stent sold under the trade name CYPHER® is widely used for the prevention of restenosis in the U.S. and abroad.

In VSMCs, it has been shown that rapamycin treatment inhibits VSMC proliferation and migration. This is believed to be due, at least in part, to an increase in the cyclin-dependent kinase inhibitor, p27^{Kipl} (Marx SO, et al., Circ Res, 1995, 76(3):412-7; Sun J, et al., Circulation, 2001, 103(24):2967-72; Grinspoon S, et al., J Clin Endocrinol Metab, 1996, 81(11):3861-3; Wallace AM, et al., Clin Endocrinol, 1999, 51(6):816-7). During the late-G₁ phase of the cell cycle, p27^{Kipl} protein levels decrease, which subsequently results in freeing the cyclin E/cyclin-dependent kinase 2 complex to activate transcription through the phosphorylation of the retinoblastoma protein.

Methods of obtaining and producing rapamycin are known in the art, and any such methods may be used to obtain or make rapamycin for use in accordance with the present invention. For example, U.S. Patent Number 3,929,992 describes methods of obtaining rapamycin by culturing a rapamycin-producing organism in an aqueous nutrient medium. Rapamycin can salso been synthesized in its naturally occurring enantiomeric form (see Nicolaou et al., (1993), J. Am. Chem. Soc. Vol. 115, p4419-4420; Schreiber et al. (1993), J. Am. Chem. Soc., Vol. 115, p7906-7907; Danishefsky et al., (1993), J. Am. Chem. Soc., Vol. 115, p9345-9346.

The compositions of the present invention may also comprise derivatives of rapamycin that are capable of inhibiting the proliferation of VSMCs and/or have anti-restenotic activity. For example, several rapamycin derivatives are known in the art, and can be used in accordance with the present invention. Novatis' Everolimus (RAD-001), Wyeth's Temsirolimus (CCI-779), and Ariad's AP23573 are examples of rapamycin derivatives currently being used on, or developed for use on, drug-eluting stents. Other suitable rapamycin derivatives are described in U.S. patents 5,661,156 and 5,728,710, both of which are entitled "Rapamycin Derivatives." U.S. Patent 6,677,357 entitled "Rapamycin 29-enols", U.S. patent 6,680,330 entitled "Rapamycin dialdehydes", and U.S. patent 6,884,429 entitled "Medical devices incorporating deuterated rapamycin for controlled delivery thereof" also describe rapamycin derivatives that may be used in accordance with the present invention.

An article by Xue et al. entitled "Effects of Rapamycin Derivative ABT-578 on Canine Smooth Muscle Cells and Endothelial Cell Proliferation" describes the synthesis and activity of 42-epi-(tetrazoylyl) rapamycin or "ABT-578" , a drug which has also been used in man on drug-eluting stents (Xue et al. Prelinica, Vol. 2, No. 6, (2004), p 451-455). Other rapamycin derivatives are described in Luengo et al. (1995) "Structure Activity Studies of Rapamycin Analogs: Evidence that the C-7 methoxy group is part of the effector domain positioned at the FKBP12-FRAP interface" Chem. Biol., Vol. 2, p 471-481; Chakraborty et al. (1995) "Design and synthesis of a rapamycin-based high affinity binding FKBP12 ligand" Chem. Biol. Vol. 2, p157-161; published U.S. patent application 2003/0129215 entitled "Medical Devices Containing Rapamycin Analogs" and U.S. patent 6,015,815 entitled "Tetrazole-containing rapamycin analogs with shortened half-lives." Fermentation and purification of rapamycin and 30-demethoxy rapamycin has been described in the literature (C. Vezina et al., J. Antibiot. (Tokyo) (1975), Vol. 28 (10), p 721; S. N. Sehgal et al., J. Antibiot. (Tokyo), 1975,28(10), 727; 1983, 36(4), 351; N. L. Pavia et al., J. Natural Products, 1991, 54(1), 167-177).

Other derivatives of rapamycin include the mono- and di-ester derivatives of rapamycin (see WO 92/05179), 27-oximes of rapamycin (see EP 0467606), 42-oxo analogs of rapamycin (see U.S. Pat. No. 5,023,262); bicyclic rapamycins (see U.S. Pat. No. 5,120,725); rapamycin dimers (see U.S. Pat. No. 5,120,727); silyl ethers of rapamycin (see U.S. Pat. No. 5,120,842); and arylsulfonates and sulfamates (see U.S. Pat. No. 5,177,203).

All of the above, and any other derivatives of rapamycin that are capable of inhibiting one or more of (a) VSMC proliferation, (b) intimal hyperplasia, (c) vascular inflammation, (d) vascular stenosis, (e) vascular restenosis, or (f) the function or signaling of mTOR, are within the scope of the present invention. One of skill in the art can readily determine whether any given rapamycin derivative will be useful according to the present invention, for example by testing its effect on VSMC proliferation and/or intimal, hyperplasia using the *in vitro* and *in vivo* biological assays described in the Examples section of this application.

In addition, other inhibitors of mTOR (a serine/threonine protein kinase) that are capable of inhibiting one or more of (a) VSMC proliferation, (b) intimal hyperplasia, (c) vascular inflammation, (d) vascular stenosis, or (e) vascular restenosis, may be used in place of rapamycin or rapamycin derivatives in the compositions, methods, and devices of the present invention. One of skill in the art can readily determine whether any given mTOR inhibitor will be useful according to the present invention, for example by testing its effect on VSMC proliferation and/or intimal hyperplasia using the *in vitro* and *in vivo* biological assays described in the Examples section of this application.

### PI3 Kinase Inhibitors

In certain aspects, the present invention provides compositions comprising a phosphatidylinositol 3-kinase ("PI3-kinase") inhibitor in combination with (a) rapamycin, (b) a leptin inhibitor, or (c) both a rapamycin and a leptin inhibitor. In other aspects the present invention provides methods of treating or preventing vascular stenosis or vascular restenosis comprising administration of such compositions and intraluminal devices coated with such compositions.

In one embodiment, the PI3-kinase inhibitor is wortmannin (CAS 19545-26-7), or a derivative thereof that has P13-kinase inhibitory function. Wortmannin is a steroidal furan of the viridian class and is a natural product of *Penicillium wortmanni.* Wipf et al. ((2005), "Chemistry and Biology of Wortmannin" Org. Biomol. Chem., 2005, Vol. 3, p 2053-2061) describe the isolation of wortmannin from *Penicillium wortmanni,* and various schemes that can be used to synthesize wortmannin. Wipf *et al.,* also describe the mechanism of PI3-kinase inhibition, the wortmannin binding site on PI3-kinase, the structures and activities of steroidal furans that are closely related to wortmannin, and structural analogs of wortannin that have PI-3 kinase inhibiting activity.

Wipf *et al.,* provide the structures of the wortmannin-related furnaosteroids (+)-halenaquinol, (+)-halenaquinone, (+)-xestoquinone, viridian, viridol, demethpxyviridin, demethoxyviridol, wortmannolone, and noelaquinone, the structures of which are reproduced below.

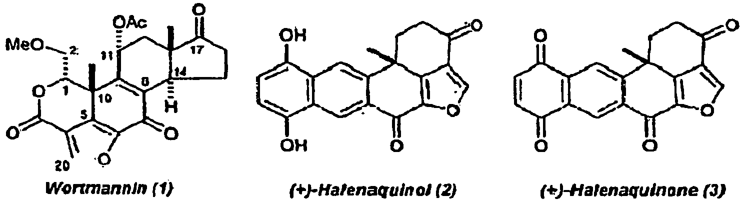

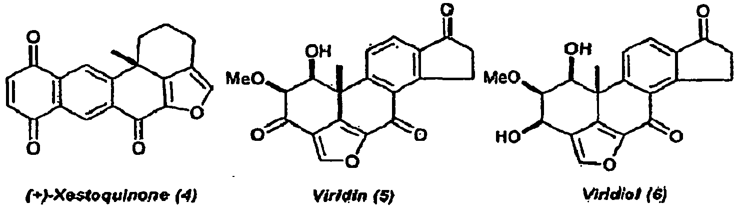

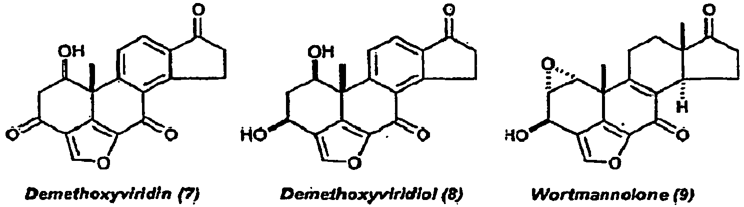

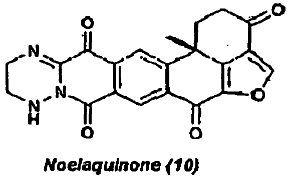

Wipf *et al.* also provide the structure of wortmannin (1) and the PI3-kinase inhibiting wortmannin analogs 2-8 as illustrated below (IC₅₀ values are IC50s for PI3-kinase inhibition):

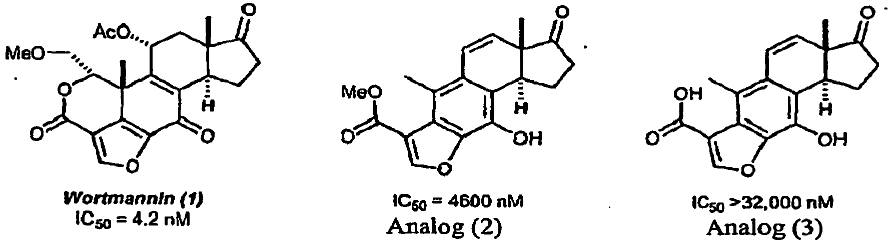

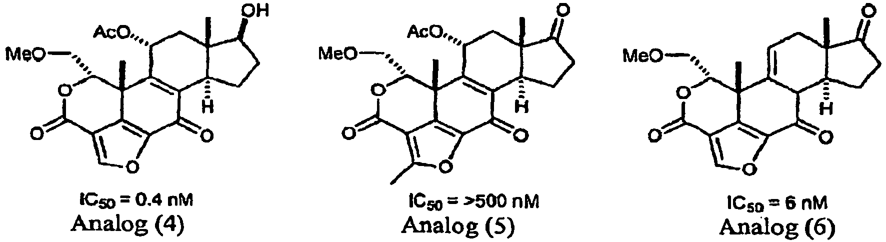

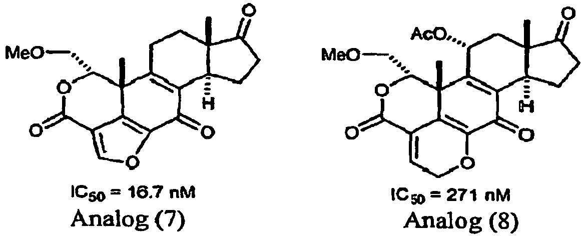

The above, and any other derivatives of wortmannin that are capable of inhibiting PI3-kinase, and/or that are capable of inhibiting one or more of (a) VSMC proliferation, (b) intimal hyperplasia, (c) vascular inflammation, (d) vascular stenosis, or (e) vascular restenosis, are within the scope of the present invention.

In another embodiment, the PI3-kinase inhibitor is 2-(4-morpholinyl)-8-phenyl-4H-1-benzopyran-4-one or "LY294002", or a derivative thereof that has PI3-kinase inhibitory function, and/or that is capable of inhibiting one or more of (a) VSMC proliferation, (b) intimal hyperplasia, (c) vascular inflammation, (d) vascular stenosis, or (e) vascular restenosis. LY294002 is commercially available from multiples sources, such as Jena Bioscience GmBH (Germany), Cell Signalling Technolgy (U.S.A), and BioSource International Inc. (U.S.A). The structure, synthesis, and PI3-kinase inhibiting activity of LY294002 are described in Vlahos et al., (1994) "A specific inhibitor of phosphatidylinositol 3-kinase, 2-(4-morpholinyl)-8-phenyl-4H-1-benzopyran-4-one (LY294002)", J. Biol. Chem. Vol. 269 p. 5241-5248; Walker et al., (2000) "Structural Determinants of PI3-kinase inhibition by Wortmannin, LY294002, Quercitin, Myricetin, and Staurosporine. Mol. Cell. Vol. 6, p909; Semba et al., (2002), "The in vitro and in vivo effects of 2-(4-morpholinyl)-8-phenyl-chromone (LY294002), a specific inhibitor of PI3-kinase, in human colon cancer cells., Clin. Cancer. Re. Vol. 8, p1957.

In yet another embodiment, the PI3-kinase inhibitor is quercitin, myricetin, or staurosporine, or a derivative of quercitin, myricetin, or staurosporine that has PI3-kinase inhibitory activity and/or that is capable of inhibiting one or more of (a) VSMC proliferation, (b) intimal hyperplasia, (c) vascular inflammation or inflammatory response, (d) vascular stenosis, or (e) vascular restenosis.

One of skill in the art can readily determine whether any given PI3-kinase inhibitor will be useful according to the present invention, for example by testing its effect on VSMC proliferation and/or intimal hyperplasia using the *in vitro* and *in vivo* biological assays described in the Examples section of this application.

### Intraluminal Devices

In certain aspects, the present invention provides intraluminal devices that are either (a) coated with the compositions of the invention, or (b) are otherwise capable of releasing the compositions of the invention within the lumen of a blood vessel. In further embodiments, the present invention provides methods of treatment and/or prevention of stenosis or restenosis comprising delivery of such intraluminal devices to subjects in need thereof.

As used herein the term "intraluminal device" refers to any medical device that that may be delivered intraluminally into a blood vessel of a subject, including, but not limited to stents, shunts, catheters, arterio-venous grafts, by-pass grafts, other types of vascular grafts, balloons for use in PTCA procedures, and any other devices or components of devices that may be inserted into the lumen of a blood vessel.

In preferred embodiments, the intraluminal devices are implantable intraluminal devices, i.e. devices that remain inside the body for an extended period of time, typically days, months, or years, such as stents. However, in other embodiments, the intraluminal devices of the invention might not be implanted. For example, the devices may be devices that are introduced into the vascular lumen for a short period of time, typically minutes or hours, such as catheters and angioplasty balloons and the like. For example, a catheter or angioplasty balloon may be used to deliver one or more doses of the compositions of the invention directly to the vicinity of a vascular stenosis.

In preferred embodiments the intraluminal devices of the invention are stents. Any type of vascular stent may be used in accordance with the present invention, such as any metal stent, polymer stent, polymer-coated stent, biodegradable stent, and the like.

The compositions of the invention can either be applied onto or into a pre-manufactured intraluminal device, or incorporated into the device itself during its manufacture. For example, if the intraluminal device is a polymeric stent or a biodegradable stent, the compositions of the invention may be added to the polymer or biodegradable material from which the stent is manufactured. It is preferred that the compositions of the invention are formulated such that they are released from the device gradually over time. In situations where the compositions are to be applied to a pre-manufactured intraluminal device, the composition may be used to coat the entire device, or a section of the device, or may be inserted into receptacles or micropores within the device.

Methods for applying drug compositions to intraluminal devices such as stents are well known in the art, and any suitable known method may be used. For example, suitable methods of applying drug compositions, including rapamycin, to stents are described in U.S. patent 6,153,252, U.S. patent, U.S. patent 6,776,796, U.S. patent 6,585,764, U.S. patent 6,273, 913, U.S. patent 6,273,913, U.S. patent 6,585,764, U.S. patent 6,120,536, and U.S. patent application 2005/0187607.

In preferred embodiments, the compositions of the invention are applied to a pre-manufactured intraluminal device. For example, the compositions of the invention may be applied to the intraluminal device (such as a polymer-coated device) by dipping the device into a solution (such as an aqueous solution) or suspension containing the compositions of the invention for a sufficient period of time (such as, for example, five minutes) and then drying the coated device, preferably by means of air drying for a sufficient period of time (such as, for example, 30 minutes). Alternatively, the compositions of the invention may be mixed with a polymer that will be used to coat the device, and applied by dipping the device the polymer mixture for a sufficient period of time (such as, for example, five minutes) and then drying the polymer-coated device, preferably by means of air drying for a sufficient period of time (such as, for example, 30 minutes).

The ideal coating material should be able to adhere strongly to the intraluminal device (in the case of stents, both before and after expansion), be capable of retaining the drug at a sufficient load level to obtain the required dose, be able to release the drug in a controlled way over a period of several days, weeks, or months, and be as thin as possible so as to minimize the increase in profile of the device caused by application of the coating. In addition, an ideal coating material should not contribute to any adverse response by the body (i.e., should be non-thrombogenic, non-inflammatory, etc.).

In some embodiments, the compositions of the invention may be added to reservoirs or micropores in the intraluminal device. A coating or membrane of biocompatable material may be applied over the reservoirs to control the diffusion of the drug from the reservoirs to the blood vessel wall. There are may methods known in the art for applying compounds to intraluminal devices such as stents, including dipping methods, spraying methods, and the like, and any such method may be used in accordance with the present invention.

In preferred embodiments, the compositions of the invention are applied to, or form part of, a polymeric coating. The polymeric coating may serve as a controlled release vehicle for the active agents in the compositions, or may act as a reservoir for the active agents. The polymeric coating may be hydrophilic, hydrophobic, biodegradable, or non-biodegradable. Any suitable polymeric materials can be used, including, but not limited to, polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyurethanes, silicones, polyorthoesters, polyanhydrides, polycarbonates, polypropylenes, polylactic acids, polyglycolic acids, polycaprolactones, polyhydroxybutyrate valerates, polyacrylamides, polyethers, and mixtures and copolymers of the foregoing. Coatings prepared from polymeric dispersions such as polyurethane dispersions (BAYHYDROL, etc.) and acrylic acid latex dispersions can also be used with the compositions of the present invention.

Biodegradable polymers that can be used in accordance with the present invention include, but are not limited to, poly(L-lactic acid), poly(DL-lactic acid), polycaprolactone, poly(hydroxy butyrate), polyglycolide, poly(diaxanone), poly(hydroxy valerate), polyorthoester; copolymers such as poly (lactide-co-glycolide), polyhydroxy(butyrate-co-valerate), polyglycolide-co-trimethylene carbonate; polyanhydrides; polyphosphoester; polyphosphoester-urethane; polyamino acids; polycyanoacrylates; biomolecules such as fibrin, fibrinogen, cellulose, starch, collagen and hyaluronic acid; and mixtures of the foregoing. Biostable materials that are suitable for use in accordance with the present invention include, but are not limited to, polyurethane, silicones, polyesters, polyolefins, polyamides, polycaprolactam, polyimide, polyvinyl chloride, polyvinyl methyl ether, polyvinyl alcohol, acrylic polymers and copolymers, polyacrylonitrile, polystyrene copolymers of vinyl monomers with olefins (such as styrene acrylonitrile copolymers, ethylene methyl methacrylate copolymers, ethylene vinyl acetate), polyethers, rayons, cellulosics (such as cellulose acetate, cellulose nitrate, cellulose propionate, etc.), parylene and derivatives thereof; and mixtures and copolymers of the foregoing.

The compositions of the invention may be applied to, or incorporated into, intraluminal devices alone, or in combination with one or more other therapeutically effective agents, and/or one or more pharmaceutically acceptable solvents (such as aqueous or nonaqueous solvents), diluents, carriers, excipients, surfactants, adjuvants, preservatives, stabilizers, wetting agents, emulsifying agents, antibacterial agents, antifungal agents, sugars, salts, agents that promote sustained release of the active compounds, agents that facilitate or limit absorption, and the like.

### Therapeutically Effective Amounts

A therapeutically effective amount of the compositions of the invention should be applied to, or incorporated into, the intraluminal devices. As used herein, the phrase "therapeutically effective amount" means an amount of the composition sufficient cause some inhibition or decrease in one or more of the following parameters: (a) VSMC proliferation, (b) intimal hyperplasia, (c) vascular inflammation, (d) vascular stenosis, or (e) vascular restenosis.

The amount and relative ratios of the compounds of the invention that should be used can be determined by routine experimentation and optimization. For example, effective amounts can be determined by performing *in vitro* experiments, *in vivo* animal experiments, and/or clinical trials, to quantify the effect of the compounds on one or more parameters such as (a) VSMC proliferation, (b) intimal hyperplasia, (c) vascular inflammation, (d) vascular stenosis, or (e) vascular restenosis, and altering the amounts or ratios of the compounds accordingly to achieve the desired outcome. Devices may be produced with differing amounts of the compositions of the invention applied thereto, and the dosage and/or number of devices to be used may be decided by the attending physician, within the scope of sound medical judgment.

The specific therapeutically effective dose level for any particular subject will depend upon a variety of factors including the disorder being treated and the severity of the disorder; activity of the specific compound(s) employed; the specific composition employed, the rate at which the compounds are released from the device, the size of the device (such as a stent), the number of devices (such as stents), the age, body weight, general health, sex and diet of the subject; the time of administration, route of administration, and rate of excretion of the specific compound employed; the duration of the treatment; drugs used in combination with or coincidental with the specific compositions employed; and other similar factors well known in the medical arts. For example, it is well within the skill of the art to start doses of the compound at levels lower than required to achieve the desired therapeutic effect and to gradually increase the dosage until the desired effect is achieved.

For systemic delivery of the compositions of the invention, preferred doses of rapamycin are from about 0.1 to about 100 mg/kg/day, or more preferably from about 1 mg/kg/day to about 20 mg/kg/day, or more preferably from about 2 mg/kg/day to about 10 mg/kg/day. For example, a dose of from about 4 mg/kg/day to about 9 mg/kg/day, by systemtic delivery, has been shown herein to be effective when combined with the PI3-kinase inhibitor LY294002.

For systemic delivery of the compositions of the compositions of the invention, preferred doses of the PI3-kinase inhibitor LY294002 are from about 0.1 to about 100 mg/kg/day, or more preferably from about 0.1 mg/kg/day to about 20 mg/kg/day, or more preferably from about 0.5mg/kg/day to about 5 mg/kg/day. For example, a dose of about 1.2 mg/kg/day has been shown herein to be effective when combined with rapamycin at from about 4 mg/kg/day to about 9 mg/kg/day.

Doses to be used when the compositions of the invention are applied to stents, can be determined by scaling the dose from the doses shown herein to be useful systemically, and by performing routine *in vitro* and *in vivo* testing to confirm effective doses, and effective ratios of each of the components of the composition of the invention.

In certain preferred embodiments, including embodiments for systemic delivery and localized delivery on an intraluminal device such as a stent, it is preferred that a PI3-kinase inhibitor (such as LY294002) and rapamycin (or a rapamycin derivative) are used in a ratio, by weight, of about 1:2, or about 1:3, or about 1:4, or about 1:5, or about 1:6, or about 1:7, or about 1:8, or about 1:9, or about 1:10, or about 1:11, or about 1:12 (P13-kinase inhibitor:rapamycin/rapamycin derivative). For example, in one preferred embodimemt LY294002 and rapamycin are used in a ratio, by weight, of from about 1:3 to about 1:10, or more preferably still a ratio, by weight, of from about 1:4 to about 1:9 (LY294002:rapamycin).

For preferred embodiments where the compositions of the invention are to be used to impregnate an intraluminal device such as a stent, the actual amount by weight that will be applied to the device will depend on the size of the device, among other factors. For a stent in standard lengths (for example from about 8mm to about 33m in length), it is preferred that the stent is impregnated with from about 50 micrograms to about 350 micrograms of rapamycin and from about 5 micrograms to about 175 micrograms of LY294002, or equally effective amounts of other PI3 kinase inhibitors or rapamycin derivatives. By way of illustration, if another PI3-kinase inhibitor is used that is has about half the potency of LY294002, approximately double the amount of that PI3-kinase inhibitor should be used, and if a rapamycin derivative is used that has about half the potency of rapamycin, approximately double the amount of that rapamycin derivative should be used.

### Subjects and Methods of Treatment

The compositions, methods and devices of the invention may be used to inhibit one or more of (a) VSMC proliferation, (b) intimal hyperplasia, (c) vascular inflammation, (d) vascular stenosis, or (e) vascular restenosis, in any subject in need thereof. For example, the subjects of the invention may be suffering from, or at risk of developing, a cardiovascular disease such as a hyperproliferative vascular disease, atherosclerosis, vulnerable plaque, coronary artery disease, peripheral artery disease, carotid artery stenosis, intimal hyperplasia, neointimal hyperplasia, vascular hyperplasia, stenosis of one or more blood vessels (i.e. "vascular stenosis"), or restenosis of one or more blood vessels after treatment (such as by PTCA or stenting) to treat or alleviate a prior stenosis, or the subjects may be suffering from, or at risk of developing, obesity, metabolic syndrome or diabetes.

The subjects may be any human or other mammalian species. In preferred embodiments, the subjects are human. In even more preferred embodiments, the subjects are humans that are afflicted with, or at risk of developing, diabetes, obesity or metabolic syndrome. Metabolic syndrome is a combination of medical disorders that increase one's risk for cardiovascular disease, obesity, and diabetes. However, it should be noted that the compositions, methods, and devices of the invention may also be used in non-obese and/or non-diabetic subjects, as it is expected that the rapamycin-combination compositions of the invention may be more useful for treating or preventing vascular stenosis than rapamycin alone, even in subjects who do not exhibit rapamycin resistance.

The subjects of the invention may be treated using a composition or medical device according to the present invention. For example, in a preferred embodiment, the subjects of the invention are treated by delivery and implantation of a stent device configured to elute the rapamycin-containing compositions of the present invention. Methods of implanting drug-eluting stents are well known by those skilled in the art, and can readily be performed by, for example, interventional cardiologists.

In other embodiments, the rapamycin-containing compositions of the invention maybe administered to subjects by means other than drug-eluting stents. For example, the rapamycin-containing compositions of the invention may be delivered locally to the vicinity of a vascular stenosis using a catheter or other intraluminal medical device. In yet further embodiments, the rapamycin-containing compositions of the invention may be administered to the subject systemically, such as by intravenous delivery, intra-arterial delivery, intransal delivery subcutanoues injection, transdermal delivery, in an oral dosage form, in any other delivery form known in the art for systemic delivery of pharmaceutical agents.

### EXAMPLES

### EXAMPLE 1

This example shows, *inter alia,* that upregulation of leptin, as occurs in diabetes and metabolic syndrome, antagonizes sirolimus-dependent inhibition of VSMC proliferation and migration by activating PI3K pathways. A murine femoral artery wire injury model of restenosis was used, and it was determined that combined therapy with an mTOR inhibitor (sirolimus) and a PI3K inhibitor (LY294002) was more effective in inhibiting restenosis than therapy with sirolimus alone.

We first assessed the effect of leptin on the proliferation of early-passage murine aortic primary VSMC. VSMC from the C57BL/6J genetic background were serum starved and subsequently treated with leptin at increasing concentrations (1, 10, 100 ng/ml) for 72 hr (Fig. 1a). Leptin increased murine VSMC proliferation in a dose-dependent fashion, compared to treatment with vehicle (Fig. 1a). Leptin-stimulated neointimal hyperplasia in mice has been shown to require the leptin receptor 14, therefore we hypothesized that the effect of leptin on VSMC proliferation would be attenuated in leptin receptor deficient mice. Indeed, leptin induced dose-dependent VSMC proliferation in leptin-deficient (ob/ob) but not in leptin receptor-defective (db/db) cells (Fig. 1b). Serum stimulation promoted VSMC proliferation equally well in ob/ob- and db/db-derived cells, suggesting that the receptor-defective db/db VSMC could respond to stimulation through other growth factors and were specifically defective in leptin-dependent signaling (Fig. 1b). These results show that leptin stimulates murine VSMC proliferation *in vitro* via the leptin receptor.

In primary murine VSMC, leptin (at a physiological concentration of 10 ng/mL) stimulated the phosphorylation of several physiological mTOR substrates: Akt (on serine 473) (Fig. 1c), S6K, and 4EBP-1 (data not shown). Leptin also stimulated the phosphorylation of the p44 and p42 forms of MAPK (Fig. 1d). Protein levels of Akt, MAPK, S6K, and 4EBP-1 did not change significantly during leptin stimulation (Fig. 1c, 1d, and data not shown). Thus, leptin can activate the MAPK and mTOR signaling pathways in cultured murine VSMC.

To determine whether MAPK or PI3K activity are important for leptin-induced proliferation of murine VSMC, we stimulated serum-starved VSMC with 100 ng/mL leptin in the absence or presence of pharmacological inhibitors to PI3K (LY294002) and MAPK kinase (U0126). Addition of 10 mM U0126, which has been reported to abolish VSMC proliferation induced by the adipokine resistin25, did not significantly alter leptin-induced proliferation (Fig. 1e). U0126 prevented leptin-stimulated phosphorylation of MAPK (data not shown). However, 10 mM LY294002, did inhibit VSMC proliferation stimulated by leptin (Fig. 1e). These results suggest that leptin-induced proliferation of murine VSMC requires PI3K activation.

We next confirmed that leptin increases neointimal hyperplasia in a murine arterial injury model, as previously reported. In the femoral artery wire injury model, neointimal hyperplasia is normally observed within 1 week after injury and peaks at 2 weeks. In our experiments, wild type C57BL/6J mice on a normal diet were randomized to receive either a sham operation (n=8), wire injury followed by vehicle treatment for 14 d (n=9), or wire injury followed by treatment with murine recombinant leptin (0.4 mg/kg daily dose) for 14 d (n=10) (Fig. 2). Compared to sham operated controls, wire injury significantly increased both the intimal area (Fig. 2a) and the intima:media (I/M) ratio (Fig. 2b a quantitative representation of neointimal formation.). A primary component of the neointima were VSMC, confirmed by immunohistochemical staining of a-actin (a protein expressed in differentiated smooth muscle, data not shown). Media areas were used to adjust for artery size. Leptin increased neointimal formation after wire injury roughly threefold (Fig. 2). Leptin levels measured 3 hr after intraperitoneal injection reached 70 ng/nL, a level 15 times that of untreated WT mice and comparable to the level (90 ng/nl) of untreated leptin receptor-defective db/db mice (data not shown). These results indicate that leptin promotes neointimal hyperplasia after arterial injury in this murine model.

To investigate the possibility that leptin may oppose the inhibitory effect of sirolimus on neointimal hyperplasia, we tested the combined effect of leptin and increasing concentrations of sirolimus on neointimal formation in the murine femoral artery injury model. Mice were randomized to treatment with: sirolimus (1 mg/kg, n=8); sirolimus (4 mg/kg, n=8); sirolimus (9 mg/kg, n=7); leptin (0.4 mg/kg) and sirolimus (1mg/kg, n=8); leptin (0.4 mg/kg) and sirolimus (4 mg/kg, n=8); or leptin (0.4 mg/kg) and sirolimus (9 mg/kg, n=7), each daily for 14 days (Fig. 2). Sirolimus at 4 and 9 mg/kg/d has been shown to inhibit VSMC migration from WT murine aortic explants29. In the absence of leptin, sirolimus significantly decreased neointimal formation (Fig. 2b), -2-fold at the lowest dose (1 mg/kg/d) compared to vehicle, -5-fold at the intermediate dose (4 mg/kg/d), and highest doses (9 mg/kg/d). Leptin significantly increased neointimal formation despite 1 mg/kg/d or 4 mg/kg/d sirolimus. Indeed, only the highest dose (9 mg/kg/d) of sirolimus resulted in inhibition of leptin stimulated neotintimal formation (Fig. 2b).

The efficacy of an inhibitor, such as sirolimus, in preventing neointimal formation was determined using the formula (1-I/Minh/ I/Mno inh), or 1 minus the I/M ratio with inhibitor divided by the I/M ratio without inhibitor, which we termed the relative neointimal inhibition (RNI). An RNI value of 1 represents total inhibition of neointimal formation. RNI values were corrected for the higher levels of neointimal formation caused by leptin in the absence of inhibitor. Without leptin (Fig. 2b), the RNI values for 1, 4, and 9 mg/kg/d sirolimus were 0.51, 0.81, and 0.85, respectively. In the presence of leptin (Fig. 2b), the RNI's for the 3 doses of sirolimus were 0.35, 0.67, and 0.97, respectively. Therefore, in the presence of exogenous leptin, sirolimus at the two lower doses (1, and 4 mg/kg/d) was significantly less effective in inhibiting neointimal formation. At the highest dose sirolimus (9 mg/kg/d) did effectively inhibit neointimal formation in the presence of exogenous leptin (Fig. 2b). These results suggest that leptin confers partial resistance to sirolimus-mediated inhibition of neointimal hyperplasia, both by increasing hyperplasia caused by arterial injury and by conferring resistance to sirolimus's inhibitory effects on neointimal formation.

Next, we examined the ability of the PI3K inhibitor LY294002, alone or in combination with sirolimus (at a dose which is partially effective in the presence of leptin), to inhibit leptin-induced neointimal formation after femoral artery injury. Mice were randomized to treatment with leptin (0.4 mg/kg) and vehicle (n=7); leptin (0.4 mg/kg), LY294002 (1.2 mg/kg), and vehicle (n=7); or leptin (0.4 mg/kg), LY294002 (1.2 mg/kg), and sirolimus (4 mg/kg, n=8) daily for 14 days (Fig. 3). Leptin-induced neointimal formation was partially inhibited by either LY294002 (1.2 mg/kg, RNI = 0.70) (Fig. 3), or sirolimus (4 mg/kg, RNI = 0.67) (Fig. 2). However, a combination of LY294002 (1.2 mg/kg) and sirolimus (4 mg/kg) achieved nearly complete inhibition of neointimal formation (RNI = 0.97) (Fig. 3). These results suggest that combined therapy with PI3K and mTOR inhibitors synergistically overcomes partial leptin-induced resistance to sirolimus following vascular injury in this murine model.

Our data may explain why the sirolimus-eluting coronary artery stent is less effective in individuals with diabetes and/or metabolic syndrome, and suggest that, stent restenosis may be more effectively treated in these individuals with combinatorial therapy, targeting both mTOR and PI3K. (PI3K inhibitor LY294002 targets the a, b, d, and g isoforms of the p110 catalytic subunit). Thus, inhibitors of PI3K, combined with mTOR inhibition, may prove effective in treating restenosis and coronary artery disease.

Figure 1 shows that leptin activates mTOR signaling in cultured murine VSMC and stimulates VSMC proliferation in a leptin receptor- and PI3K-dependent fashion. Panels (a-b, e) show VSMC Proliferation. In panels (a-b) serum starved primary aortic VSMC from wild type ("WT"), ob/ob, or db/db mice were stimulated with indicated concentrations of leptin or serum. The figure legend denotes genotypes. Triplicate experiments were quantitated and the "Control" is vehicle treated. * P value <0.05, # P value <0.001 compared to control with Dunnett's test. (c-d) Akt and MAPK phosphorylation. Serum-starved WT murine VSMCs were stimulated with leptin and subjected to immunoblotting. Densitometries of phosphorylation (normalized to total protein and untreated control) from three independent experiments are shown. * P value <0.05 compared with control. (c, inset) S473-phosphorylated Akt (Ph-Akt, upper panel) and total Akt (lower panel). Panel (d), inset, shows phosphorylated MAPK (Ph-p44 and Ph-p42, upper panel) and total MAPK (p44 and p42, lower panel). Molecular weight markers are indicated to the left in insets. Panel (e) shows the effects of LY294002 and U0126 in VSMC proliferation assay. * P value <0.001 compared to the control, # P value <0.001 compared to leptin alone.

Figure 2 shows that leptin-induced neointimal hyperplasia after arterial injury in mice is resistant to sirolimus inhibition. Panel (a) shows femoral artery injury. Micrographs of neointimal formation (arrows) in representative injured femoral arteries in the presence or absence of leptin and rapamycin are indicated. Cross-sections were stained with elastic-Van Gieson to highlight the elastic laminae in the tunica media (black rings) and collagen in the tunica adventitia. Panel (b) shows intima:media (I/M) ratios measured from femoral arteries. The white bars show sham injury. The solid black bars, show injury without leptin. The striped bars, show injury with leptin. P values after Tukey's test are indicated above brackets.

Figure 3 shows that a combination of a PI3K inhibitor (LY294002) and a mTOR inhibitor (sirolimus) inhibits leptin-induced neointimal hyperplasia in mice. Panel (a) shows data from femoral artery injury studies. Micrographs of leptin-induced neointimal formation (arrows) in injured femoral arteries after treatment with LY294002 and/or rapamycin are illustrated. Elastic-van Gieson staining was performed to highlight elastic laminae (black rings) and collagen. Panel (b) shows I/M ratios. Tukey's P values are provided over the brackets.

The methods used in the above experiments were as follows: Cell Culture and VSMC Proliferation Assays. Primary VSMC were isolated from aortic explants of wildtype, ob/ob, or db/db mice in the method of Roque *et al.*30. VSMC were routinely cultured in Dulbecco's Modified Eagle's Medium (DMEM) containing either 4500 or 1250 mg/L glucose supplemented with 20% FBS at 37°C in a 5% CO2 incubator. Experiments were performed on cells at less than 10 passages in culture and showed similar results in high and low glucose media. Cells were plated at 200,000 cells/ well of a 6 well dish and subsequently serum starved for 72 hr in DMEM media supplemented with 0.1% FBS. Leptin dissolved in PBS was added directly to serum-free media at concentrations of 1, 10, or 100 ng/ml with 10 mM LY294002 or U0126, and cells were grown for another 72 hr before harvesting. VSMC proliferation was measured by counting samples using a Coulter counter or using the Cell Titre 96 Aqueous One Solution Cell Proliferation Assay kit (Promega). Cell viability was assessed by Trypan Blue staining. Similar results were obtained with thymidine incorporation assays.

Animal Models and Drug Treatments. Wild type C57BL/6J background mice and mice deficient for leptin (C57BL/6J-Lepob; ob/ob) or the leptin receptor (C57BL/6J-m +/+ Lepdb; db/db) were purchased from Charles Jackson Laboratories, Maine. Mice were obtained at ages 6-8 weeks old and average weight 15g. Animals were fed standard rodent chow and tap water ad libitum. Procedures and animal care were approved by the Institutional Animal Care and Use Committee. Mice were subjected to femoral artery wire injury or sham injury described below 24 hr prior to starting pharmacological treatments. Sirolimus was administered by intraperitoneal injection once per day for 14 days as a suspension in 0.2% sodium carboxymethyl cellulose and 0.25% polysorbate-80; duplicate experiments were accomplished with 0.5% DMSO. Leptin or LY294002 was administered by intraperitoneal injection once per day for 14 days as a suspension in PBS. Plasma leptin levels were measured at baseline, 3, 6, and 10 hr after leptin injection using an ELISA assay (R&D). Leptin plus the vehicle used for both sirolimus and LY294002 was used as the control group for the treatment groups with sirolimus and LY294002. After the 2-week treatment, animals were processed for femoral artery morphometry.

Femoral Artery Injury Model. Endoluminal injury of bilateral femoral arteries was performed according to the method of Roque *et al.* with minor modification27. General anesthesia was achieved with an intraperitoneal injection of a mixture of Ketamine (100 mg/kg) and Xylazine (15 mg/kg). Briefly, to denude and dilate femoral arteries, a 0.014" angioplasty guidewire (Guidant) was passed endoluminally 3 times to the level of the aortic bifurcation. A sham protocol was performed where the arteries underwent dissection, clamping, and ligature without passage of the guidewire.

Computer-aided quantitative morphometry to measure luminal, medial, intimal, and vessel areas were performed as previously described27 with minor modification. Mice were euthanized via CO2 asphyxiation prior to en bloc excision of hind limbs. Briefly, multiple segments of the common femoral artery at the level of injury were fixed in 10% Zinc Formalin, embedded in paraffin, sectioned at 5 mm thickness, and subjected to Elastic-Van Gieson staining at a Columbia University Core Histology facility. In many cases, arteries underwent immunohistochemical staining for smooth muscle a-actin to highlight the media and neointima. Photomicrographs were captured on Adobe Photoshop 7.0 and transferred to Scion Image software for quantitative analysis. Specimens were analyzed by two independent operators blinded to the treatment groups. Microsoft Excel software was used to calculate the RNI values from 1/M ratios in the absence and presence of pharmacological inhibitors, as detailed in the text.

Statistical methods. Data are expressed as mean ± standard error of measurement (SEM). Multiple comparisons were made on Graphpad prism software by one-way analysis of variance (ANOVA) followed by post hoc analysis of means with Tukey's HSD test or, in the case of comparison to a control, with Dunnett's test. Statistical results are in the Supplemental Table.

### EXAMPLE 2

Reagents: Recombinant human leptin was purchased from R&D Systems (Minneapolis, MN). U0126, a specific inhibitor of MEK1/2, and LY294002, a specific phosphatidylinositol 3-kinase inhibitor were purchased from Calbiochem (La Jolla, CA). Rabbit polyclonal anti-phospho-(Thr²⁰²/Tyr²⁰⁴) p42/p44^{MAPK} and anti-p42/p44^{MAPK}, rabbit polyclonal anti-phospho-Akt(Ser⁴³⁷) and anti-Akt, rabbit anti-p70^{S6Kinase} antibodies were purchased from Cell Signaling Technology (Beverly, MA). Rabbit polyclonal anti-4EBP1 antibody was purchased from Bethyl (Montgomery, TX).

Cell Culture: Primary VSMC lines cell lines have been isolated from aortic explants of normal C57/B16 mice, as well as *ob*/*ob* and *db*/*db* mice. VSMCs are grown in Dulbecco's Modified Eagle's Medium (DMEM) containing either 4500 or 1250 mg/L glucose supplemented with 20% FBS (Invitrogen, Carlsbad, CA) at 37°C and 5% CO₂. Only cells passaged less than 12 times were used. Rapamycin and insulin were purchased from Calbiochem (San Diego, CA). Human glycated albumin was purchased from Exocell, Inc. (Philadelphia, PA), and platelet derived growth factor (PDGF) was purchased from Cell Signaling, Inc.

Proliferation of VSMCs: VSMC proliferation was measured by counting triplicate samples using a Coulter counter or using the CellTitier 96 Aqueous One Solution Cell Proliferation Assay (Promega, Madison, WI) kit per manufacturer's instructions. Cell viability was assessed via staining with Trypan Blue.

Migration of VSMCs: Migration was measured using a modified Boyden chamber as previously described (Grinspoon S, et al., J Clin Endocrinol Metab, 1996, 81(11):3861-3). The lower chambers contained PDGF (10 ng/mL), insulin (200 nmol/L), or 0.2% BSA (negative control) in DMEM. 2x10⁵ cells were loaded into the top chamber. After 6 h, non-migrating cells were scraped from the upper surface of the filter, and the lower surface of the filter was stained with HEMA 3 Stain Set (Fisher Diagnostics, Middletown, VA). The number of migrated VSMCs was determined by counting 4 fields at 200X magnification, of constant area per well. Values are expressed as the percentage of cells migrating in response to a chemo-attractant after subtracting the negative control. Experiments were performed thrice in triplicate wells.

Animal Models: C57BL/6J mice and mice deficient for leptin (C57BL/6J-Lep^{ob}; *ob*/ob) or the leptin-receptor (C57BL/6J-m +/+ Lep^{db}; *db*/*db*) (6-8 weeks old, average weight about 15g) were purchased from the Charles Jackson Laboratories and housed in the facilities of the Division for Comparative Medicine at the Columbia University Medical Center, New York, NY. The mice were fed standard rodent chow and tap water *ad libitum.* Procedures and animal care were approved by the Institutional Animal Care and Use Committee and were in accordance with the Guide for the Care and Use of Laboratory Animals (7th ed. 1996, Washington, D.C.: National Academy Press, 125).

Surgical Procedures: The mouse femoral artery wire injury model of was used with minor modifications (Roque M, et al., Arterioscler Thromb Vasc Biol, 2000, 20(2):335-42). Briefly, forty-two male mice were studied. Endoluminal injury to the common femoral artery was produced by either 3 passages of a 0.014" diameter angioplasty guidewire (Guidant, Santa Clara, CA). General anesthesia was achieved with an intraperitoneal injection of a mixture of Ketamine (100 mg/kg) and Xylazine (15 mg/kg). While being viewed under a surgical microscope, a groin incision was made and the femoral artery was isolated and temporarily clamped distal to the inguinal ligament. An arteriotomy was performed and the guidewire was passed 3 times to the level of the aortic bifurcation. The artery was then ligated proximal to the arteriotomy. A sham protocol was carried out on the contralateral side where the arteries underwent dissection, temporary clamping, and ligature, without passage of the wire. The formation of the neointima was conformed in this model as being comprised mainly of VSMCs through immunohistochemical staining of representative sections for α-smooth muscle actin via a commercial kit (Sigma).

### Treatment Protocol I

For the rapamycin dose response study, mice were randomized into 4 treatment groups: Vehicle (n=5), 1 mg/kg/d rapamycin (n=5), 4 mg/kg/d rapamycin (n=6), and 9 mg/kg/d rapamycin (n=4).

### Treatment Protocol II

For the studies carried out in hyperglycemic mice, animals received an intraperitoneal injection of 60 mg/kg/d for 5 days of streptozotocin (STZ) in 0.05 mol/L citrate buffer. Surgery was performed 3 days following the final injection. Blood glucose levels were measured prior to injury (representing baseline levels), at time of injury, at euthanasia, and as needed during insulin administration with the OneTouch SureStep ® Blood glucose monitoring system (Lifescan, Inc., Milipitas, CA). Only animals with blood glucose concentrations > 300 mg/dL were included in this study. Mice were randomized into 4 groups: hyperglycemic (STZ, n=7), hyperglycemic with insulin treatment (STZ + INS, n=4), hyperglycemic with rapamycin treatment (STZ + RPM, n=6), and hyperglycemic with both insulin and rapamycin treatment (STZ + INS + RPM, n=5).

For all treatment protocols, rapamycin was administered by intraperitoneal injection once per day, for 14 days, as a suspension in 0.2% sodium carboxymethyl cellulose and 0.25% polysorbate-80. Leptin was administered by intraperitoneal injection once per day, for 14 days, as a suspension in 0.9% saline. Insulin (12-26 U/kg) was administered via an intraperitoneal route twice per day, for 14 days, according to blood glucose measurements.

### Treatment Protocol III

For the studies in leptin induced neointimal growth, mice were randomized into 6 treatment groups: Vehicle (n=6), 0.4 mg/kg/d leptin (n=6), 0.4 mg/kg/d leptin with 4 mg/kg/d rapamycin (n=6), 0.4 mg/kg/d leptin with 9 mg/kg/d rapamycin (n=6). 0.4 mg/kg/d leptin with 1.2 mg/kg/d LY294002, and 0.4 mg/kg/d leptin with 4 mg/kg/d rapamycin plus 1.2 mg/kg/d LY294003.

Morphometry: Mice were euthanized via CO₂ asphyxiation and were perfused with 10% Zinc Formalin through the left ventricle at physiological pressure (100 mm Hg). Tissues were excised and fixed overnight in 10% Zinc Formalin, embedded in paraffin, and sectioned at 5 µm thickness. Multiple level sections were cut at 100 µm intervals. For morphometry, tissues were stained with elastic van Gieson stain. Using computer aided morphometry (Scion Image), luminal, medial, intimal, and vessel area, and the lengths of the internal and external elastic laminae were measured as previously described (Roque M, et al., Arterioscler Thromb Vasc Biol, 2000, 20(2):335-42) in a blinded manner.

Western blot analysis: The cells were plated in a 100-mm culture dish in DMEM supplemented with 20% FBS for 24 h at 37°C, followed by serum starvation for 48 h. Cells were then treated with different reagents for 30 min. Cells were subsequently lysed on ice for 30 min in RIPA buffer [150mM NaCl, 100mM Tris (pH 8.0), 1% Triton X-100, 1% deoxycholic acid, 0.1% SDS, 5mM EDTA, and 10mM NaF] supplemented with 1mM sodium vanadate, 2mM leupeptin, 2mM aprotinin, 1mM phenylmethylsulfonyl fluoride (PMSF), 1mM DTT, and 2mM pepstatin A. After centrifugation at 14,000 rpm for 30 min, the supernatant was harvested as the total cellular protein extract, and stored at -70°C. The protein concentration was determined using Bio-Rad protein assay reagent (Richmond, CA). The total cellular protein extracts were separated by SDS-polyacrylamide gel electrophoresis (SDS-PAGE) and transferred to nitrocellulose membrane in 20mM Tris-HCl (pH 8.0) containing 150mM glycine and 20% (v/v) methanol.

Membranes were blocked with 5% nonfat dried milk in PBS containing 0.05% Tween 20 and incubated with antibodies against phospho-(Thr²⁰²/Tyr²⁰⁴) p42/p44^{MAPK} and p42/p44^{MAPK}, phospho-Akt(Ser⁴³⁷) and anti-Akt, p70^{S6K}, 4EBP-1. Blots were washed three times in PBS buffer, followed by incubation with the appropriate HRP-linked IgG. The specific proteins in the blots were visualized using an enhanced chemiluminescence reagent (NEN, Boston, MA).

Statistical methods: All data are expressed as mean ± SEM. One-way ANOVA analysis was performed using the GraphPad Prism 4 software package. When an overall significant difference was observed, Tukey's HSD test was used to compare the individual mean values.

### Rapamycin inhibits VSMC proliferation in the presence of insulin stimulation.

As insulin is known to stimulate VSMC proliferation, the ability of rapamycin to inhibit the insulin-induced proliferation of VSMCs was measured. Insulin (10 or 100 nmol/L) induced a dose dependent increase in cell proliferation (FIG. 4). Co-incubation with rapamycin and insulin blocked the insulin-stimulated proliferation in a dose dependent manner with the 10nmol/L rapamycin concentration yielding a significant (p<0.01) decrease.

### Rapamycin inhibits VSMC migration toward insulin.

Insulin has been shown to possess chemo-attractant properties toward VSMCs (Indolfi C, et al., Circulation, 2001, 103(24):2980-6) and pre-treatment, but not acute treatment, of VSMCs with rapamycin for 48 hours inhibits migration toward the chemo-attractant PDGF (Grinspoon S, et al., J Clin Endocrinol Metab, 1996, 81(11):3861-3). The ability of rapamycin to inhibit insulin- and PDGF-induced migration of VSMCs was examined. Pre-treatment with rapamycin inhibited VSMC migration induced by insulin and PDGF to a similar extent (FIG. 5). Surprisingly, acute rapamycin treatment inhibited insulin-induced migration, in addition to pre-treatment with rapamycin. The ability of an acute rapamycin exposure to block VSMC migration illustrates a difference in rapamycin's effect on insulin-mediated VSMCs migration compared to that of the PDGF-mediated event, suggesting that the chemoattractant effect mediated by insulin requires the PI3K/Akt/mTOR pathway.

### Leptin stimulates VSMC proliferation in a dose dependent manner with independence of glucose level.

Hyperleptinemia is associated with diabetes, obesity and/or metabolic syndrome. The effect of various concentrations of leptin on the proliferation of murine VSMCs grown in DMEM with high (4500 mg/L) and normal (1250 mg/L) glucose concentrations was assessed. Leptin potentiated the proliferation of VSMCs in a dose-dependent manner, in cells exposed to both high and normal glucose concentrations (FIG.6A). VSMCs from *ob*/*ob* mice (animals unable to produce leptin) and *db*/*db* mice (animals that harbor an impaired leptin receptor) were used. Leptin receptor-impaired mice served as controls. Experiments show that leptin can induce VSMC proliferation in cells obtained from *ob*/*ob* mice, but not from *db*/*db* mice (FIG. 6A and 6B).

### Rapamycin inhibits leptin stimulated VSMC proliferation.

To evaluate the role of rapamycin in leptin-induced VMSC proliferation, murine VSMCs cultured in DMEM were co-incubated with various concentrations of rapamycin and 100 ng/ml of leptin. As shown in FIG. 7A, rapamycin completely inhibited the leptin-induced VSMC proliferation, even at a low dosage (10 nmol/L).

### Leptin activates both MEK/ERK 1/2 and PI3/Akt pathways.

In order to further explore the signal transduction pathways involved in leptin stimulated VSMC proliferation, the effects of leptin on Akt and ERK1/2 activation were investigated (FIG. 8). Both ERK1/2 and Akt were rapidly and transiently phosphorylated after leptin (100 ng/ml) stimulation, reaching a maximum at 10 min and decreasing thereafter at 45 min. Rapamycin at a high concentration (100 mM) did not inhibit the phosphorylation of either ERK1/2 or Akt.

### Rapamycin abolishes the leptin-induced phosphorylation of p70^{S6K} and 4E-BP1.

The study described in this example provides the first evidence that p70^{S6K} and 4E-BP1 are up-phosphorylated after leptin stimulation (FIG. 9). As compared with serum-stimulated phosphorylation of p70^{S6K} and 4E-BP1, leptin at 100 ng/ml stimulated a minor migration of the phosphorylated bands. Rapamycin, when co-incubated with leptin, completely inhibited the up-phosphorylation both in p70^{S6K} and 4E-BP1. This indicates that the mTOR pathway is involved in leptin-stimulated VSMC proliferation. When the above-stated phosphorylation results were combined with the cell proliferation data, which showed that rapamycin even at low concentrations completely abolished leptin-induced VSMC proliferation; the mTOR appears to play a vital role in leptin-mediated VSMC proliferation.

### Rapamycin resistance in VSMC with the combination of leptin and insulin stimulation.

Since neither insulin nor leptin alone showed any resistance to rapamycin effects, the potential mechanism underlying elevated restenosis rates post-angiography in patients with diabetes and/or obesity was examined. The combination effects of the two elevated hormone levels may be the cause of rapamycin resistance. In cultured VSMCs, co-incubation with leptin (100 ng/ml) and insulin (10 nmol/L) lead to ~80% proliferation, while rapamycin, even at 1 µM, cannot inhibit VSMC proliferation (FIG. 7B). To further clarify the mechanism, a specific MEK/ERK1/2 inhibitor, U0126, and a specific PI3K inhibitor, LY294002, were co-incubated with or without leptin, insulin and rapamycin. Compared to the effect of rapamycin alone on VSMC proliferation, U0126, when co-incubated with or without rapamycin, results in a weak inhibition of VSMC proliferation that is induced by a combination treatment of leptin and insulin. LY294002 exposure has a more pronounced effect in inhibiting VSMC proliferation that is induced by a combination treatment of rapamycin, leptin, and insulin than 100 nmol/L of rapamycin alone. LY294002 completely inhibits VSMC proliferation induced by leptin and insulin when co-incubated with 100 nmol/L of rapamycin (FIG. 7C). Thus, it is suggested that the PI3K/Akt pathway, especially upstream of mTOR, might be a key site of rapamycin resistance induced by a combination of elevated leptin and insulin.

### Rapamycin treatment inhibits neointimal formation in the mouse wire injury model of vascular injury in a dose-dependent manner.

The ability of rapamycin to inhibit neointimal formation in the mouse wire injury model was assessed via utilizing various rapamycin concentrations. As such, an appropriate median dose was determined for further studies. The femoral arteries in 20 mice were injured using three passes of a 0.014" guide wire as previously described (Roque M, et al., Arterioscler Thromb Vasc Biol, 2000, 20(2):335-42). Mice then received 1, 4, or 9 mg/kg/d of rapamycin (via an intraperitoneal route) or vehicle for 14 days. The data demonstrated that rapamycin treatment induced a dose-dependent decrease in the intima media ratio and intimal area with 4 and 9 mg/kg/d resulting in a significant decrease in neointimal formation (p < 0.01). No significant differences were observed in the medial areas of these sections. Data from these experiments disclose 4 mg/kg/d as a median dose for further studies in assessing the ability of rapamycin to inhibit neointimal hyperplasia in other mouse models.

### Rapamycin is effective in inhibiting neointimal formation in hyperglycemic mice and hyperglycemic mice receiving insulin.

The effects of hyperglycemia and insulin treatment on the ability of rapamycin to inhibit neointimal formation were next examined. A separate set of 22 mice received an intraperitoneal injection of streptozotocin (STZ, 60 mg/kg/d) 5 days prior to wire-injury. This STz treatment induced hyperglycemia, wherein high serum glucose levels are defined as blood glucose levels > 300 mg/dl. The animals then were subjected to femoral wire injury and were treated for 14 days with 4 mg/kg/d of rapamycin administered via an intraperitoneal route. A subset of those animals also received 12-26 U/kg of insulin twice per day according to their blood glucose measurements. This dose has been reported previously to stimulate an increase in neointimal hyperplasia following balloon injury in the streptozotocin-induced hyperglycemic rat (Indolfi C, et al., Circulation, 2001, 103(24):2980-6). Administration of this insulin dose in these mice was found to reduce blood glucose levels to normal levels for several hours, and co-administration of rapamycin did not alter its effect.

At 14 days, animals were euthanized and the arteries were harvested and processed for morphometry. Hyperglycemia alone (STZ) did not alter neointimal formation. However, hyperglycemia coupled with insulin treatment (STZ + INS) induced a significant increase in the intima:media ratio (p<0.01) (FIG. 10). Furthermore, rapamycin was effective in reducing neointimal formation in both hyperglycemic mice (STZ + RPM) and hyperglycemic mice receiving insulin (STZ + INS + RPM). These data suggest that hyperglycemia alone and hyperglycemia coupled with insulin treatment do not alter regulation of neointimal hyperplasia by the rapamycin-mediated mTOR pathway.

### Leptin enhances the neointirnal formation in the mouse wire injury model and rapamycin treatment inhibit the neointimal formation only in the high dose group.

To investigate whether hyperleptinemia can enhance neointimal formation, twelve mice were randomly separated into a vehicle group (n=6) and a leptin treatment group (n=6). After femoral artery wire injury, mouse recombinant leptin (0.4 mg/kg/d) or vehicle were given intraperitoneally once per day for 14 days. Both the intimal area and intimal:media ratio were significantly increased in the leptin treatment group. To further test the effect of rapamycin on leptin-induced neointimal formation, twelve mice were randomly treated with leptin (0.4 mg/kg/d) and rapamycin (4 mg/kg/d) (n=6) or rapamycin alone (9 mg/kg/d) (n=6) for 14 days after femoral artery wire injury. However, the median rapamycin dose can only partially inhibit leptin-induced neointimal growth, whereas a high dose of rapamycin can completely inhibit neointimal growth to the level of sham surgery (FIG. 11). This result is indicative of a potential resistance to rapamycin. The effect of LY294002 on cells exhibiting leptin-stimulated neointimal formation was also examined. Twelve mice were randomly treated with leptin (0.4 mg/kb/d) and LY294002 (1.2 mg/kg/d) (n=6), or with leptin (0.4 mg/kb/d), LY294002 (1.2 mg/kg/d), and rapamycin (4 mg/kg/d) (n=6) for 14 days after femoral artery wire. The LY294002 plus rapamycin group but not the LY294002 alone group revealed a complete inhibition of neointimal hyperplasia induced by exogenous leptin. These data corroborate cell culture experiments where LY294002 was introduced to cells exhibiting leptin-stimulated neointimal formation.

Figure 4 is a bar graph showing rapamycin inhibition of insulin-stimulated VSMC (vascular smooth muscle cell) proliferation. Murine VSMCs (2 X 10⁴ cells/well) were incubated in a 6-well tissue culture plate for 72 hours in DMEM containing 0.1 % FBS with or without insulin (10 or 100 nmol/L) and with 0, 1, 10, or 100 nmol/L rapamycin. Triplicate samples were counted using a Coulter counter and were normalized as percentage of the control group. Cell viability was checked via Trypan blue staining. Brackets indicate significant (P<0.01) differences.

Figure 5 is a graph that depicts inhibition of migration of VSMCs toward insulin by rapamycin. Effects of acute and 48h exposure of VSMCs to rapamycin (RPM, 100 nmol/L) on migration toward PDGF (10 ng/mL) and Insulin (200 nmol/L) in a modified Boyden chamber. # indicates value is significant compared to control group, P<0.01.

Figure 6 illustrates that leptin stimulates VSMC proliferation in a dose-dependent manner in the presence of both high and low glucose concentrations. In panel A, leptin stimulates VSMC proliferation in wild type mice displaying high glucose levels (450 mg/dL), wild type mice displaying normal glucose levels (125 mg/dL) and in *ob*/*ob* mice. In panel B, leptin had no effect on VSMC proliferation in cells from the *db*/*db* mouse, compared with 10% of FBS (serum). Murine VSMCs, seeded 2 X 10⁴ cells/well in a 6-well plate, were starved for 72 hrs in DMEM with 0.1 % FBS. Leptin in concentrations of 1, 10, and 100 ng/ml were added to the culture for 72 hrs. Triplicate samples were counted via a Coulter counter and were normalized as percentage of the control group. Cell viability was checked via Trypan blue staining. * indicates a significance value of P<0.05 compared to control samples; # indicates a significance value of P<0.01 compared to control samples.

Figure 7 shows that rapamycin inhibits leptin-stimulated VSMC proliferation but not VSMC proliferation stimulated by a combination of insulin and leptin. Murine VSMCs, seeded 2 X 10⁴ cells/well in a 6-well plate, were starved for 72 hrs in DMEM with 0.1% FBS. Rapamycin, leptin, or insulin in different concentrations indicated in the figure were incubated with VSMCs in DMEM with 0.1% of FBS for 72 hrs. Triplicate samples were counted via a Coulter counter. Cell viability was checked via Trypan blue staining. In panel A, rapamycin completely inhibits leptin-induced VSMC proliferation. In panel B, rapamycin cannot completely inhibit VSMC proliferation when cells were stimulated with a combination of leptin and insulin. In panel C, VSMCs were exposed to U0126, a specific MEK1/2 inhibitor, or LY294002, a specific PI3K inhibitor. Cells were incubated with or without rapamycin, leptin (100 ng/mL), and insulin (10 nmol/L) as indicated in the figure. VSMCs exposed to LY294002 and treated with 100 nmol/L of rapamycin exhibited an inhibition in VSMC proliferation induced by a combination of leptin and insulin treatment. # indicates that the value was significant compared with the control group, P<0.01.

Figure 8 is a western blot showing that leptin stimulates phosphorylation of ERK1/2 and Akt. Cells were starved and incubated with leptin (100ng/ml). Samples were then collected at different time points as indicated. In panel A, whole cell extracts were analyzed by immunoblotting using an anti-phosphorylated- ERK1/2 antibody (upper panel) or anti-ERKl/2 (lower panel). In panel B, the whole cell extracts were analyzed by immnoblotting using an anti-phosphorylated-Akt (Ser437) antibody (upper panel) or anti-Akt antibody (lower panel).

Figure 9 is a western blot that depicts rapamycin completely dephosphorylating 4E-BP1 and p70^{S6K}, proteins that are phosphorylated by leptin and serum in the absence of rapamycin. Starved murine VSMCs were incubated for 20 min with leptin (100 ng/ml) or 10% FBS, in the absence or presence of rapamycin (100 nmol/L). Whole cell extracts were analyzed by western blot with anti-4E-BP1 (upper panel) and anti-P70^{S6K} (lower panel) antibodies. The mobility of phosphorylated 4E-BP1 and P70^{S6K} proteins is reduced on SDS-gel.

Figure 10 is a graph illustrating that rapamycin inhibits neointimal formation in the femoral artery following wire injury in a hyperglycemic mouse model and a hyperglycemic mouse model in the presence of insulin. Wire injury lesions in Streptozotocin-induced diabetic mice were divided into five groups, sham operation group (sham), hyperglycemic without treatment (STZ), hyperglycemic with insulin treatment (STZ + INS), hyperglycemic with rapamycin treatment (STZ + RPM), and hyperglycemic with insulin and rapamycin treatment (STZ + INS + RPM). Rapamycin treatment was administered as a single intraperitoneal injection of 4 mg/kg/d and insulin treatment was a twice-daily injection of 12-26 U/kg according to blood glucose. The animals were sacrificed after 14 days and the femoral arteries were processed and stained as described in the methods section. The intimal area (panel A), media area (panel B), and intima:media ratio (panel C) were measured via computer aided morphometry. # indicates that values were significant to P<0.01 when compared with the sham group.

Figure 11 shows that leptin enhances neointimal hyperplasia in wire injured femoral artery and that it is partially inhibited by rapamycin at 4 mg/kg/d. Histologic sections are shown in the top panel. Mice that underwent femoral artery surgery were divided into seven groups: (A) sham, (B) wire injury, (C) wire injury with leptin 0.4 mg/kg/d, (D) wire injury with leptin 0.4 mg/kg/d and rapamycin 4 mg/kg/d, (E) wire injury with leptin 0.4 mg/kg/d and rapamycin 9 mg/kg/d, (F) wire injury with leptin 0.4 mg/kg/d and LY294002 1.2 mg/kg/d, and (G) wire injury with leptin 0.4 mg/kg/d and rapamycin 4 mg/kg/d plus LY294002 1.2 mg/kg/d. After a 14-day treatment, the animals were sacrificed and the femoral arteries were processed and stained as described in the methods section. The upper panel shows the representative micrographs of the femoral arteries of different groups described above. Bars in the graph represent 10 µm. The lower panel shows the intimal area (panel H), media area (panel I), and intima:media ratio (panel J). Values were measured via a computer-aided morphometry program. # indicates significance value when compared with the sham group, P<0.01.

Figure 12 is a bar graph of leptin serum concentrations in mice. Blood samples from STZ-treated and *db*/*db* mice that were administered an intraperitoneal injection of leptin (0.4 mg/kg) were collected 3, 6 and 10 hours after injection. Blood samples were collected to determine baseline serum leptin levels in wild type mice prior to treatment with STZ. The serum concentrations of leptin were measured with a mouse leptin TiterZyme EIA kit (Ann Arbor, Michigan).Leptin stimulates VSMC proliferation independent of glucose levels *in vitro* and rapamycin completely reverses that action. However, resistance to the inhibitory effects of rapamycin is observed in VSMC proliferation when cells grown in culture are incubated in the presence of both leptin and insulin. Additionally, VSMC proliferation induced by a combination treatment of leptin and insulin is inhibited when a PI3K specific inhibitor, LY294002, in conjunction with rapamycin are used to treat VSMCs grown in culture. However, VSMC proliferation was not inhibited in the presence of the ERK1/2 specific inhibitor, U0126.

Exogenous leptin in the femoral artery wire injury mouse model stimulates significant neointimal hyperplasia wherein a high dose of rapamycin (9 mg/kg/d) or rapamycin (4 mg/kg/d) in conjunction with LY294002 inhibit proliferation of neointimal cells. These data further support that a hyperglycemic state and insulin treatment do not contribute to a loss of rapamycin efficacy in diabetic patients. Rather, the data uphold that hyperleptinemia and insulin resistance may be key factors contributing to higher ISR rates in metabolic syndrome/diabetic patients. As such, the combination treatment of rapamycin and a PI3-kinase inhibtor, such as LY294002, may be useful for treatment or prevention of restenosis in diabetic patients. Leptin inhibitors may also be useful.

### EXAMPLE 3

A solution of rapamycin, and either (a) a PI3-kinase inhibitor, or (b) a leptin inhibitor, or (c) both a PI3-kinase inhibitor and a leptin inhibtor, may be prepared in a solvent miscible with a polymer carrier solution, and mixed with the polymer to give a final concentration of each drug in polymer mixture in the range 0.0001 % w/w to 30 % w/w. (w/w denotes the mass of the drug in the polymer mixture as a percentage of the mass of the entire mixture). The polymer solution should be selected such that it is biocompatible (i.e., such that it will not elicit any negative tissue reaction or promote thrombus formation *in vivo*). The polymer should ideally also be degradable, such as a lactone-based polyester or copolyester, e.g., a polylactide, a polycaprolacton-glycolide, a polyorthoester, a polyanhydride; a poly-aminoacid; a polysaccharide; a polyphosphazene; a poly(ether-ester), or a blend thereof. Non-absorbable biocompatible polymers are also suitable candidates. Polymers such as polydimethylsiolxane, poly(ethylene-vingylacetate), acrylate based polymers or copolymers, e.g., poly(hydroxyethyl methylmethacrylate, polyvinyl pyrrolidinone; fluorinated polymers such as polytetrafluoroethylenem, or cellulose esters could be used.

The polymer/drug mixture may be applied to the surface of a stent by either dip-coating, spray coating, brush coating, or some other method, and the solvent allowed to dry to leave a film with entrapped rapamycin and either (a) a PI3-kinase inhibitor, or (b) a leptin inhibitor, or (c) both a PI3-kinase inhibitor and a leptin inhibtor.

### EXAMPLE 4

A stent, whose body contains micropores or channels may be dipped into a solution of rapamycin and either (a) a PI3-kinase inhibitor, or (b) a leptin inhibitor, or (c) both a PI3-kinase inhibitor and a leptin inhibtor, wherein the concentration of each drug in the solution is in the range 0.0001 wt % to saturated, in an organic solvent such as acetone or methylene chloride, for sufficient time to allow the solution to permeate into the pores. After the solvent has been allowed to dry, the stent may be dipped briefly in fresh solvent to remove excess surface bound drug. A solution of a polymer, such as any of those identified Example 2, may be applied to the stent to form an outerlayer of polymer that will act as diffusion-controller for release of the drugs from the stent.

## Claims

1. An intraluminal device for use in the treatment or prevention of vascular stenosis or restenosis, wherein the intraluminal device is impregnated with, and configured to release, a therapeutically effective amount of (i) an mTOR inhibitor, and (ii) a PI3-kinase inhibitor, with the therapeutically effective amount ranging from 50 micrograms to 350 micrograms of rapamycin and from 5 micrograms to 175 micrograms of LY294002, or equally effective amounts of other mTOR inhibitors or P13 kinase inhibitors based on their relative potency to rapamycin or LY294002.

2. The interluminal device of claim 1, wherein the PI3-kinase inhibitor and the mTOR inhibitor are present in a ratio, by weight, of 1:2, 1:3, 1:4, 1:5, 1:6, 1:7, :1:8, 1:9, 1:10, 1:11, 1:12 or 1:3 to 1:10.

3. The intraluminal device of claim 1, wherein the P13-kinase inhibitor and the mTOR inhibitor are present in a ratio by weight, of 1:2 or 1:3 to 1:10 or 1:11 or 1:12.

4. The intraluminal device according to any preceding claim, wherein the mTOR inhibitor is selected from the group consisting of: rapamycin (sirolimus), everolimus (RAD-001), temsirolimus (CCI-779), AP23573, ABT-578, 42-epi-(tetrazoylyl) rapamycin, 30-demethoxy rapamycin, a rapamycin 29-enol, a tetrazole-containing rapamycin derivative, a mono-ester derivative of rapamycin, a di-ester derivative of rapamycin, a 27-oxime derivative of rapamycin, a 42-oxo derivative of rapamycin, a bicyclic derivative of rapamycin, a rapamycin dimer, a silyl ether derivative of rapamycin, an arylsulfonate derivative of rapamycin, and a sulfamate derivative of rapamycin.

5. The intraluminal device according to any one of the preceding claims, wherein the PI3-kinase inhibitor is selected from the group consisting of: wortmannin, wortmannin analogue 2, wortmannin analogue 3, wortmannin analogue 4, wortmannin analogue 5, wortmannin analogue 6, wortmannin analogue 7, wortmannin analogue 8, (+)-halenaquinol, (+)-halenaquinone, (+)-xestoquinone, viridian, viridol, demethpxyviridin, demethoxyviridol, wortmannolone, noelaquinone, 2-(4-morpholinyl)-8-phenyl-4H-1-benzopyran-4-one, LY294002, quercitin, myricetin, and staurosporine, and derivatives thereof.

6. Use of a composition in the preparation of an intraluminal device for the treatment or prevention of vascular stenosis or restenosis, wherein the composition comprises a therapeutically effective amount of (i) an mTOR inhibitor, and (ii) a PI3-kinase inhibitor, with the therapeutically effective amount ranging from 50 micrograms to 350 micrograms of rapamycin and from 5 micrograms to 175 micrograms of LY294002, or equally effective amounts of other mTOR inhibitors or P13 kinase inhibitors based on their relative potency to rapamycin or LY294002.

7. The use of claim 5, wherein the PI3-kinase inhibitor and the mTOR inhibitor are present in a ratio, by weight, of 1:2, 1:3, 1:4, 1:5, 1:6, 1:7, :1:8, 1:9, 1:10, 1:11, 1:12 or 1:3 to 1:10.

8. The use of claim 5, wherein the P13-kinase inhibitor and the mTOR inhibitor are present in a ratio by weight, of 1:2 or 1:3 to 1:10 or 1:11 or 1:12.

9. The use according to claim 6, 7, or 8, wherein the mTOR inhibitor is selected from the group consisting of: rapamycin (sirolimus), everolimus (RAD-001), temsirolimus (CCI-779), AP23573, ABT-578, 42-epi-(tetrazoylyl) rapamycin, 30-demethoxy rapamycin, a rapamycin 29-enol, a tetrazole-containing rapamycin derivative, a mono-ester derivative of rapamycin, a di-ester derivative of rapamycin, a 27-oxime derivative of rapamycin, a 42-oxo derivative of rapamycin, a bicyclic derivative of rapamycin, a rapamycin dimer, a silyl ether derivative of rapamycin, an arylsulfonate derivative of rapamycin, and a sulfamate derivative of rapamycin.

10. The use according to one of claims 6 to 9, wherein the PI3-kinase inhibitor is selected from the group consisting of: wortmannin, wortmannin analogue 2, wortmannin analogue 3, wortmannin analogue 4, wortmannin analogue 5, wortmannin analogue 6, wortmannin analogue 7, wortmannin analogue 8, (+)-halenaquinol, (+)-halenaquinone, (+)-xestoquinone, viridian, viridol, demethpxyviridin, demethoxyviridol, wortmannolone, noelaquinone, 2-(4-morpholinyl)-8-phenyl-4H-1-benzopyran-4-one, LY294002, quercitin, myricetin, and staurosporine, and derivatives thereof.

11. The use according to any of claims 6 to 10, wherein the intraluminal device is a stent designed to be administered intravascularly.

## Patentansprüche

1. Eine intraluminale Vorrichtung zur Verwendung bei der Behandlung von oder Vorbeugung gegen Gefäßstenose oder Restenose, wobei die intraluminale Vorrichtung imprägniert ist und konfiguriert ist, das sie eine therapeutisch wirksame Menge (i) eines mTOR-Inhibitors und (ii) eines P13-Kinase-Inhibitors freisetzt, wobei die therapeutisch wirksame Menge zwischen 50 Mikrogramm und 350 Mikrogramm Rapamycin und 5 Mikrogramm und 175 Mikrogramm LY294002 oder gleichermaßen wirksamen Mengen anderer mTOR-Inhibitoren oder P13-Kinase-Inhibitoren basierend auf deren relativer Stärke i. Vgl. zu Rapamycin oder LY294002 liegt.

2. Die intraluminale Vorrichtung entsprechend Anspruch 1, wobei der P13-Kinase-lnhibitor und der mTOR-Inhibitor in einem Gewichtsverhältnis von 1:2, 1:3, 1:4, 1:5, 1:6 1:7, 1:8, 1:9, 1:10, 1:11, 1:12 oder 1:3 bis 1:10 vorhanden sind.

3. Die intraluminale Vorrichtung entsprechend Anspruch 1, wobei der P13-Kinase-lnhibitor und der mTOR-Inhibitor in einem Gewichtsverhältnis von 1:2 oder 1:3 bis 1:10 oder 1:11 oder 1:12 vorhanden sind.

4. Die intraluminale Vorrichtung entsprechend einem der vorhergehenden Ansprüche, wobei der mTOR-Inhibitor aus der Gruppe bestehend aus Folgenden ausgewählt wird: Rapamycin (Sirolimus), Everolimus (RAD-001), Temsirolimus (CCI-779), AP23573, ABT-578, 42-Epi-(tetrazolyl)-Rapamycin, 30-Demethoxy-Rapamycin, einem Rapamycin-29-Enol, einem Tetrazol-haltigem Rapamycin-Derivat, einem Monoesterderivat von Rapamycin, einem Diesterderivat von Rapamycin, einem 27-Oximderivat von Rapamycin, einem 42-Oxoderivat von Rapamycin, einem bicyclischen Derivat von Rapamycin, einem Rapamycin-Dimer, einem Silyletherderivat von Rapamycin, einem Arylsulfonatderivat von Rapamycin und einem Sulfamatderivat von Rapamycin.

5. Die intraluminale Vorrichtung entsprechend einem der vorhergehenden Ansprüche, wobei der P13-Kinase-lnhibitor aus der Gruppe bestehend aus Folgenden ausgewählt wird: Wortmannin, Wortmannin-Analog 2, Wortmannin-Analog 3, Wortmannin-Analog 4, Wortmannin-Analog 5, Wortmannin-Analog 6, Wortmannin-Analog 7, Wortmannin-Analog 8, (+)-Halenaquinol, (+)-Halenaquinon, (+)-Xestoquinon, Viridian, Viridol, Demethoxyviridin, Demethoxyviridol, Wortmannolon, Noelaquinon, 2-(4-Morpholinyl)-8-Phenyl-4H-1-Benzopyran-4-on, LY294002, Quercitin, Myricetin und Staurosporin und Derivate dieser.

6. Die Verwendung einer Zusammensetzung in der Erstellung einer intraluminalen Vorrichtung zur Behandlung von oder Vorbeugung gegen Gefäßstenose oder Restenose, wobei die Zusammensetzung eine therapeutisch wirksame Menge (i) eines mTOR-Inhibitors und (ii) eines P13-Kinase-lnhibitors enthält, wobei die therapeutisch wirksame Menge zwischen 50 Mikrogramm und 350 Mikrogramm Rapamycin und 5 Mikrogramm und 175 Mikrogramm LY294002 oder gleichermaßen wirksamen Mengen anderer mTOR-Inhibitoren oder P13-Kinase-lnhibitoren basierend auf deren relativer Stärke i. Vgl. zu Rapamycin oder LY294002 liegt.

7. Die Verwendung entsprechend Anspruch 5, wobei der P13-Kinase-lnhibitor und der mTOR-Inhibitor in einem Gewichtsverhältnis von 1:2, 1:3, 1:4, 1:5, 1:6 1:7, 1:8, 1:9, 1:10, 1:11, 1:12 oder 1:3 bis 1:10 vorhanden sind.

8. Die Verwendung entsprechend Anspruch 5, wobei der P13-Kinase-lnhibitor und der mTOR-Inhibitor in einem Gewichtsverhältnis von 1:2 oder 1:3 bis 1:10 oder 1:11 oder 1:12 vorhanden sind.

9. die Verwendung entsprechend Anspruch 6,7 oder 8, wobei der mTOR-Inhibitor aus der Gruppe bestehend aus Folgenden ausgewählt wird: Rapamycin (Sirolimus), Everolimus (RAD-001), Temsirolimus (CCI-779), AP23573, ABT-578, 42-Epi-(tetrazolyl)-Rapamycin, 30-Demethoxy-Rapamycin, einem Rapamycin-29-Enol, einem Tetrazol-haltigem Rapamycin-Derivat, einem Monoesterderivat von Rapamycin, einem Diesterderivat von Rapamycin, einem 27-Oximderivat von Rapamycin, einem 42-Oxoderivat von Rapamycin, einem bicyclischen Derivat von Rapamycin, einem Rapamycin-Dimer, einem Silyletherderivat von Rapamycin, einem Arylsulfonatderivat von Rapamycin und einem Sulfamatderivat von Rapamycin.

10. Die Verwendung entsprechend einem der Ansprüche 6 bis 9, wobei der P13-Kinase-Inhibitor aus der Gruppe bestehend aus Folgenden ausgewählt wird: Wortmannin, Wortmannin-Analog 2, Wortmannin-Analog 3, Wortmannin-Analog 4, Wortmannin-Analog 5, Wortmannin-Analog 6, Wortmannin-Analog 7, Wortmannin-Analog 8, (+)-Halenaquinol, (+)-Halenaquinon, (+)-Xestoquinon, Viridian, Viridol, Demethoxyviridin, Demethoxyviridol, Wortmannolon, Noelaquinon, 2-(4-Morpholinyl)-8-Phenyl-4H-1-Benzopyran-4-on, LY294002, Quercitin, Myricetin und Staurosporin und Derivate dieser.

11. Die Verwendung entsprechend einem der Ansprüche 6 bis 10, wobei die intraluminale Vorrichtung ein Stent ist, der für intravaskuläre Verabreichung gedacht ist.

## Revendications

1. Un dispositif intraluminal destiné à une utilisation dans le traitement ou la prévention de resténose ou sténose vasculaire, où le dispositif intraluminal est imprégné avec, et configuré de façon à libérer, une quantité thérapeutiquement efficace de (i) un inhibiteur de mTOR, et (ii) un inhibiteur de kinase P13, avec la quantité thérapeutiquement efficace allant de 50 microgrammes à 350 microgrammes de rapamycine et de 5 microgrammes à 175 microgrammes de LY294002, ou des quantités efficaces de manière égale d'autres inhibiteurs de mTOR ou inhibiteurs de kinase P13 en fonction de leur puissance relative à rapamycine ou LY294002.

2. Le dispositif interluminal selon la Revendication 1, où l'inhibiteur de kinase P13 et l'inhibiteur de mTOR sont présents dans un rapport, en poids, de 1:2, 1:3, 1:4, 1:5, 1:6, 1:7, 1:8, 1:9, 1:10, 1:11, 1:12 ou de 1:3 à 1:10.

3. Le dispositif intraluminal selon la Revendication 1, où l'inhibiteur de kinase P13 et l'inhibiteur de mTOR sont présents dans un rapport, en poids, de 1:2 ou 1:3 à 1:10 ou 1:11 ou 1:12.

4. Le dispositif intraluminal selon l'une quelconque des Revendications précédentes où l'inhibiteur de mTOR est sélectionné dans le groupe se composant de : rapamycine (sirolimus), évérolimus (RAD-001), temsirolimus (CCI-779), AP23573, ABT-578, 42-épi-(tétrazoylyl) rapamycine, 30-déméthoxy rapamycine, une rapamycine 29-énol, un dérivé de rapamycine contenant tétrazole, un dérivé de rapamycine monoester, un dérivé de rapamycine diester, un dérivé de rapamycine 27-oxime, un dérivé de rapamycine 42-oxo, un dérivé de rapamycine bicyclique, un dimère de rapamycine, un dérivé de rapamycine d'éther de silyle, un dérivé de rapamycine arylesulfonate et un dérivé de rapamycine sulfamate.

5. Le dispositif intraluminal selon l'une quelconque des Revendications précédentes, où l'inhibiteur de kinase P13 est sélectionné dans le groupe se composant de : wortmannine, analogue de wortmannine 2, analogue de wortmannine 3, analogue de wortmannine 4, analogue de wortmannine 5, analogue de wortmannine 6, analogue de wortmannine 7, analogue de wortmannine 8, (+)-halénaquinol, (+)-halénaquinone, (+)-xestoquinone, viridian, viridol, déméthoxyviridine, déméthoxyviridol, wortmannolone, noélaquinone, 2-(4-morpholinyle)-8-phényle-4H-1-benzopyrane-4-one, LY294002, quercitine, myricétine et staurosporine, et des dérivés de ceux-ci

6. L'utilisation d'une composition dans la préparation d'un dispositif intraluminal pour le traitement ou la prévention de resténose ou sténose vasculaire, où la composition contient une quantité thérapeutiquement efficace de (i) un inhibiteur de mTOR, et (ii) un inhibiteur de kinase P13, avec la quantité thérapeutiquement efficace allant de 50 microgrammes à 350 microgrammes de rapamycine et de 5 microgrammes à 175 microgrammes de LY294002, ou des quantités efficaces de manière égale d'autres inhibiteurs de mTOR ou inhibiteurs de kinase P13 en fonction de leur puissance relative à rapamycine ou LY294002.

7. L'utilisation selon la Revendication 5, où l'inhibiteur de kinase P13 et l'inhibiteur de mTOR sont présents dans un rapport, en poids, de 1:2, 1:3, 1:4, 1:5, 1:6, 1:7, 1:8, 1:9, 1:10, 1:11, 1:12 ou 1:3 à 1:10.

8. L'utilisation selon la Revendication 5, où l'inhibiteur de kinase P13 et l'inhibiteur de mTOR sont présents dans un rapport, en poids, de 1,2 ou 1:3 à 1:10 ou 1:11 ou 1:12.

9. L'utilisation selon la Revendication 6, 7 ou 8, où l'inhibiteur de mTOR est sélectionné dans le groupe se composant de : rapamycine (sirolimus), évérolimus (RAD-001), temsirolimus (CCI-779), AP23573, ABT-578, 42-épi-(tétrazoylyl) rapamycine, 30-déméthoxy rapamycine, une rapamycine 29-énol, un dérivé de rapamycine contenant tétrazole, un dérivé de rapamycine monoester, un dérivé de rapamycine diester, un dérivé de rapamycine 27-oxime, un dérivé de rapamycine 42-oxo, un dérivé de rapamycine bicyclique, un dimère de rapamycine, un dérivé de rapamycine d'éther de silyle, un dérivé de rapamycine arylsulfonate et un dérivé de rapamycine sulfamate.

10. L'utilisation selon l'une quelconque des Revendications 6 à 9, où l'inhibiteur de kinase P13 est sélectionné dans le groupe se composant de : wortmannine, analogue de wortmannine 2, analogue de wortmannine 3, analogue de wortmannine 4, analogue de wortmannine 5, analogue de wortmannine 6, analogue de wortmannine 7, analogue de wortmannine 8, (+)-halénaquinol, (+)-halénaquinone, (+)-xestoquinone, viridian, viridol, déméthoxyviridine, déméthoxyviridol, wortmannolone, noélaquinone, 2-(4-morpholinyle)-8-phényle-4H-1-benzopyrane-4-one, LY294002, quercitine, myricétine et staurosporine, et des dérivés de ceux-ci

11. L'utilisation selon l'une quelconque des Revendications 6 à 10, où le dispositif intraluminal est une endoprothèse vasculaire conçue de façon à être administrée par voie intravasculaire.
